# FASCICULE DE BREVET EUROPEEN

(11) **EP 1 680 395 B1**
(45) Date de publication et mention de la délivrance du brevet: **25.05.2011**
(21) Numéro de dépôt: 04791557.4
(22) Date de dépôt: 15.10.2004
(51) Int. Cl.: C07C 251/48, C07D 207/32

(54) **NOUVEAUX LIGANDS ACTIVATEURS DES RECEPTEURS RARs, UTILISATION EN MEDECINE HUMAINE AINSI QU'EN COSMETIQUE**
NEUE RAR REZEPTOR AKTIVIERENDE LIGANDEN UND DEREN VERWENDUNG IN DER HUMANMEDIZIN UND DER KOSMETIK
NOVEL LIGAND ACTIVATING RAR RECEPTORS USED IN HUMAN MEDICINE AND COSMETICS

(30) Priorité: 17.10.2003 FR 0312154
(43) Date de publication de la demande: 19.07.2006
(73) Titulaire: GALDERMA RESEARCH & DEVELOPMENT, 06410 Biot (FR)
(72) Inventeur: BIADATTI, Thibaud, F-06650 Opio (FR); LUZY, Anne-Pascale, F-06560 Valbonne (FR)
(74) Mandataire: Allab, Myriam
(86) Numéro de dépôt international: PCT/FR2004/002646
(87) Numéro de publication internationale: WO 2005/037772

(56) Documents cités:
- EP-A- 0 816 352
- WO-A-01/43712
- WO-A-94/15901
- GB-A- 2 246 777
- US-B1- 6 395 260
- Thomas L. Lemke: "Review of organic functional groups" 1988, LEA & Febiger , Philadelphia, USA , page 2,3,23,3
- Michael B. Smith and J. March: "March's advanced organic chemistry, 5th edition" 2001, Wiley Interscience , New York , pages 363-364

## Description

L'invention se rapporte, à titre de produits industriels nouveaux et utiles, à de nouveaux composés, ligands activateurs des récepteurs RARs. Elle se rapporte également à des compositions les contenant, et à leur utilisation dans des compositions pharmaceutiques destinées à un usage en médecine humaine ou vétérinaire, ou bien encore dans des compositions cosmétiques et à l'utilisation non thérapeutique de ces dernières.

Les composés ayant une activité de type rétinoïde (vitamine A et ses dérivés) sont largement décrits dans la littérature, comme ayant des activités dans les processus de prolifération et différentiation cellulaire. Ces propriétés confèrent à cette classe de composés, un fort potentiel dans le traitement ou la prévention de nombreuses pathologies, et plus particulièrement en dermatologie et les cancers. Beaucoup d'effets biologiques des rétinoïdes sont médiés par la modulation des récepteurs nucléaires de l'acide rétinoique (RAR).

Les récepteurs RARs activent la transcription en se liant à des éléments de séquences d'ADN, appelés les éléments de réponse des RAR Element (RARE), sous forme d'un hétérodimère avec les récepteurs X des rétinoïdes (appelés les RXRs).

Trois sous-types de RARs humains ont été identifiés et décrits : les RARα, RARβ et RARγ.

Il est connu de l'art antérieur des composés chimiques ayant une activité activatrice des récepteurs de type RAR. On peut citer notamment les composés biaryl hétérocycliques aromatiques décrits dans le brevet EP 0 816 352 B1 qui trouvent des applications dans le traitement des affections dermatologiques, rhumatismales, respiratoires et ophtalmologiques ainsi que dans le domaine cosmétique.

Il n'en demeure pas moins que le désir de traiter des affections humaines, notamment dermatologiques, ou d'embellir cosmétiquement la peau conduit toujours à la recherche incessante de nouveaux agents actifs.

La demanderesse a maintenant découvert, de manière surprenante et inattendue de nouveaux composés hétérocycliques qui sont des ligands activateurs des récepteurs de l'acide rétinoique et qui trouvent des applications en médecine humaine, notamment en dermatologie, et dans le domaine de la cosmétique.

Ainsi, la présente invention concerne des composés répondant à la formule générale suivante : 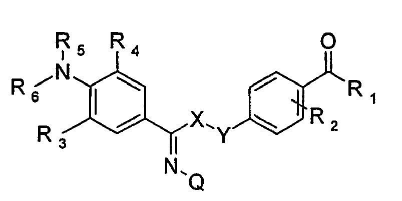 dans laquelle :
- R1 représente un radical -OH, un radical OR7, ou un radical -NR8R9 ; R7, R8 et R9 ayant les significations données ci-après,
- R2 représente un atome d'hydrogène, un atome de fluor, un atome de chlore, un radical alkyle de 1 à 3 atomes de carbone, un radical -OH, un radical -OR10,
   R10 ayant les significations données ci-après,
- R3 représente un atome d'hydrogène, un radical méthyle, un radical éthyle, un radical isopropyle, un radical tertiobutyle, un radical -CF3 ;
- R4 représente un atome d'hydrogène, un atome de fluor, un atome de chlore, un radical -OR11, un radical méthyle, un radical éthyle, un radical isopropyle, un radical tertiobutyle, ou un radical -CF3 ;
   R11 ayant les significations données ci-après ;
- R5, R6 identiques ou différents, représentent un atome d'hydrogène, un radical méthyle, un radical éthyle, un radical n-propyle, un radical isopropyle, un radical tertiobutyle, un radical -COR12 ou peuvent former ensemble un groupement piperidine, pyrrolidine, morpholine, pyrrolidin-2-one, ou piperidin-2-one, substitué ou non par un ou plusieurs groupements méthyle, éthyle, fluor, ou chlore ;
   R12 ayant les significations données ci-après,
- Q représente un radical -OH, un radical -OR13 ;
   R13 ayant les significations données ci-après ;
- X-Y représente une liaison de structure suivante : 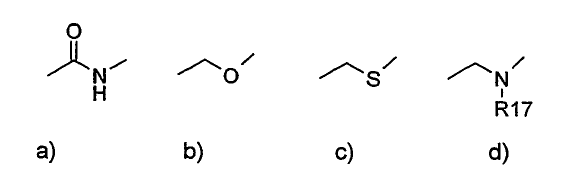 R7 ayant les significations données ci-après,
- R7 représente un radical alkyle, linéaire ou ramifié, ayant de 1 à 20 atomes de carbone, un radical alkényle, un radical monohydroxyalkyle, un radical polyhydroxyalkyle, un radical aryle, un radical aralkyle éventuellement substitué, ou un reste de sucre.
- R8, R9 identiques ou différents, représentent un atome d'hydrogène, un radical -OH, un radical alkyle de 1 à 6 atomes de carbone, un radical monohydroxyalkyle, un radical polyhydroxyalkyle, un radical aryle éventuellement substitué, forment avec l'azote de la structure générale un reste d'aminoacide ou un reste de peptide, ou bien R8 et R9, pris ensemble, forment un groupement pipéridine, pyrrolidine, morpholine, pyrrolidin-2-one ou piperidin-2-one;
- R10 représente un radical alkyle ayant de 1 à 6 atomes de carbone;
- R11 représente un radical alkyle ayant de 1 à 6 atomes de carbone, ou un radical mono ou polyéther ;
- R12 représente un radical alkyle ayant de 1 à 6 atomes de carbone ou un groupement -OR18 ;
   R18 ayant les significations données ci-après,
- R13 représente un radical alkyle ayant de 1 à 15 atomes de carbone, linéaire ou ramifié ;
- R17 représente un atome d'hydrogène, un radical alkyle ayant de 1 à 6 atomes de carbone, un radical -CF3 ;
- R18 représente un radical alkyle ayant de 1 à 6 atomes de carbone ;
   et les sels des composés de formule (I) lorsque R₁ représente une fonction OH ainsi que les isomères optiques et géométriques desdits composés de formule (I).

Lorsque les composés selon l'invention se présentent sous forme d'un sel, il s'agit de préférence d'un sel d'un métal alcalin ou alcalino-terreux, ou encore de zinc ou d'une amine organique.

Selon la présente invention, on entend par alkyle ayant de 1 à 6 atomes de carbone de préférence les radicaux méthyle, éthyle, n-propyle, i-propyle, c-propyle, isopropyle, n-butyle, i-butyle, t-butyle, n-pentyle ou n-hexyle.

Par alkyle, linéaire ou ramifié, ayant de 1 à 15 atomes de carbone, on entend notamment les radicaux méthyle, éthyle, propyle, isopropyle, cyclopropyle, n-butyle, i-butyle, t-butyle, n-pentyle, n-hexyle, 2-éthyl-hexyle, octyle ou dodécyle. Lorsque le radical alkyle est en ayant de 1 à 20 atomes de carbone, on entend en outre les radicaux hexadécyle ou octadécyle.

Par monohydroxyalkyle, on entend un radical ayant de préférence 1 à 6 atomes de carbone, notamment un radical hydroxyméthyle, 2-hydroxyéthyle, 2-hydroxypropyle ou 3-hydroxypropyle.

Par polyhydroxyalkyle, on entend un radical ayant de préférence 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical polyéther, on entend un radical ayant de 2 à 6 atomes de carbone interrompu par au moins deux atomes d'oxygène tel que les radicaux méthoxyméthoxy, méthoxyéthoxy ou méthoxyéthoxyméthoxy.

Par radical aryle, on entend de préférence un radical phényle éventuellement substitué par au moins un halogène, un alkyle ayant de 1 à 6 atomes de carbone, un hydroxyle, un alkoxy en C₁-C₃, une fonction nitro, un radical polyéther ou une fonction amino éventuellement protégée par un groupe acétyle ou éventuellement substitué par au moins un alkyle ayant de 1 à 6 atomes de carbone ou un alkoxy ayant de 1 à 6 atomes de carbone.

Par radical aralkyle, on entend de préférence le radical benzyle ou phénéthyle éventuellement substitué par au moins un halogène, un alkyle ayant de 1 à 6 atomes de carbone, un hydroxyle, un radical alkoxy ayant de 1 à 3 atomes de carbone, une fonction nitro, un radical polyéther ou une fonction amino éventuellement protégée par un groupe acétyle ou éventuellement substitué par au moins un alkyle ayant de 1 à 6 atomes de carbone ou un radical alkoxy ayant de 1 à 6 atomes de carbone.

Par radical alkényle, on entend un radical ayant de préférence 2 à 5 atomes de carbone et présentant une ou plusieurs insaturations éthyléniques, tel que plus particulièrement le radical allyle.

Par reste de sucre, on entend un reste dérivant notamment du glucose, du galactose ou du mannose, ou bien encore de l'acide glucuronique tel que 6'-mannosyl, 6'-glucosyl, 6'-galactosyl.

Par reste d'aminoacide, on entend notamment un reste dérivant de la lysine, de la glycine ou de l'acide aspartique, et par reste de peptide on entend plus particulièrement un reste de dipeptides ou de tripeptides résultant de la combinaison d'acides aminés.

Selon la présente invention, les composés de formule (I) plus particulièrement préférés sont ceux pour lesquels l'une au moins, et de préférence toutes les conditions ci-dessous sont respectées :
- R1 représente le radical -OR7 ;
- R2 représente un hydrogène ou un -OH ;
- R5, R6 identiques ou différents représentent un radical méthyle ou un radical éthyle ou forment ensemble un groupement pyrrolidine ;
- Q représente un radical -OR13.

Parmi les composés de formule (I) entrant dans le cadre de la présente invention, on peut notamment citer les composés suivants :
1. Acide 4-[2-(3-*tert-*butyl-4-diethylamino-phenyl)-2-hydroxyimino-ethoxy]-benzoïque ;
2. Acide 4-[2-(3-*tert*-butyl-4-diethylamino-phenyl)-2-hydroxyimino-ethoxy]-2-hydroxy-benzoïque ;
3. Acide 4-[2-(3-*tert*-butyl-4-pyrrolidin-1-yl-phenyl)-2-hydroxyimino-ethoxy]-benzoïque ;
4. Acide 4-[2-(3-*tert*-butyl-4-pyrrolidin-1-yl-phenyl)-2-hydroxyimino-ethoxy]-2-hydroxy-benzoïque ;
5. Acide 4-{2-[4-(acetyl-ethyl-amino)-3-*tert*-butyl-phenyl]-2-hydroxyimino-ethoxy}-benzoïque ;
6. Acide 4-{2-[4-(acetyl-ethyl-amino)-3-*tert*-butyl-phenyl]-2-hydroxyimino-ethoxy}-2-hydroxy-benzoïque ;
7. 4-[2-(3-*tert*-Butyl-4-diethylamino-phenyl)-2-hydroxyimino-ethoxy]-2-hydroxy-benzoate d'éthyle ;
8. 4-[2-(3-*tert*-Butyl-4-diethylamino-phenyl)-2-hydroxyimino-ethoxy]-2-hydroxy-benzoate d'isopropyle ;
9. 4-[2-(3-*tert*-Butyl-4-diethylamino-phenyl)-2-hydroxyimino-ethoxy]-2-hydroxy-benzoate d'isobutyle ;
10. 4-[2-(3-*tert*-Butyl-4-diethylamino-phenyl)-2-hydroxyimino-ethoxy]-2-hydroxy-benzoate d'octyle ;
11. 4-[2-(3-*tert*-Butyl-4-diethylamino-phenyl)-2-hydroxyimino-ethoxy]-2-hydroxy-benzoate de dodecyle ;
12. 1-(3-*tert*-Butyl-4-diethylamino-phenyl)-2-[3-hydroxy-4-(morpholine-4-carbonyl)-phenoxy]-ethanone oxime ;
13. 4-[2-(3-*tert*-Butyl-4-diethylamino-phenyl)-2-hydroxyimino-ethoxy]-N-ethyl-2-hydroxy-N-methyl-benzamide ;
14. Acide 4-[2-(3-*tert*-butyl-4-diethylamino-phenyl)-2-hydroxyimino-ethylsulfanyl]-2-hydroxy-benzoïque ;
15. Acide 4-[2-(3-*tert*-butyl-4-diethylamino-phenyl)-2-hydroxyimino-ethylsulfanyl]-benzoïque ;
16. Acide 4-[2-(3-*tert*-Butyl-4-diethylamino-phenyl)-2-hydroxyimino-ethylamino]-2-hydroxy-benzoïque ;
17. Acide 4-{[2-(3-*tert*-Butyl-4-diethylamino-phenyl)-2-hydroxyimino-ethyl]-methyl-amino}-2-hydroxy- benzoïque ;
18. Acide 4-[3-(3-*tert*-Butyl-4-diethylamino-phenyl)-3-hydroxyimino-propyl]-2-hydroxy-benzoïque ;
19. Acide 4-[2-(3-*tert*-Butyl-4-diethylamino-phenyl)-2-hydroxyimino-acetylamino]-2-hydroxy-benzoïque ;
20. Acide 4-[2-(3-*tert*-Butyl-4-diethylamino-phenyl)-2-hydroxyimino-acetylamino]-benzoïque ;
21. Acide 4-[2-(3-*tert*-Butyl-4-diethylamino-phenyl)-2-methoxyimino-acetylamino]-2-hydroxy- benzoïque ;
22. Acide 4-[2-(3-*tert*-Butyl-4-diethylamino-phenyl)-2-methoxyimino-ethoxy]-2-hydroxy-benzoïque ;
23.Acide 4-[2-(3-tert-Butyl-5-chloro-4-diethylamino-phenyl)-2-hydroxyimino-ethoxy]-2-hydroxy- benzoïque ;
24. Acide 4-[2-(3-tert-Butyl -4-diethylamino-5-fluoro -phenyl)-2-hydroxyimino-ethoxy]-2-hydroxy- benzoïque ;
25. Acide 4-[2-(3-tert-Butyl-4-diethylamino-5-trifluoromethyl-phenyl)-2-hydroxyimino-ethoxy]-2-hydroxy- benzoïque ;
26. Acide 4-[2-(3-tert-Butyl -4-diethylamino-5-methyl-phenyl)-2-hydroxyimino-ethoxy]-2-hydroxy- benzoïque ;
27. Acide 4-[2-(3-tert-Butyl -4-diethylamino-5-ethyl-phenyl)-2-hydroxyimino-ethoxy]-2-hydroxy- benzoïque ;
28. Acide 4-{2-[3-tert-Butyl-4-diethylamino-5-(2-ethoxy-ethoxy)-phenyl]-2-hydroxyimino-ethoxy}-2-hydroxy- benzoïque ;
29. Acide 4-[2-(3-tert-Butyl -4-diethylamino-5-propoxy-phenyl)-2-hydroxyimino-ethoxy]-2-hydroxy- benzoïque ;
30. Acide 4-[2-(4-diethylamino-3-iso-propyl-phenyl)-2-hydroxyimino-ethoxy]-2-hydroxy-benzoïque ;
31. Acide 4-[2-(4-diethylamino-3-trifluoromethyl-phenyl)-2-hydroxyimino-ethoxy]-2-hydroxy-benzoïque ;
32. Acide 4-[2-(4-diethylamino-phenyl)-2-hydroxyimino-ethoxy]-2-hydroxy-benzoïque ;
33. Acide 4-{2-[3-tert-butyl-4-(ethyl-methyl-amino)-phenyl]-2-hydroxyimino-ethoxy}-2-hydroxy-benzoïque ;
34. Acide 4-[2-(3-*tert*-butyl-4-dimethylamino-phenyl)-2-hydroxyimino-ethoxy]-2-hydroxy-benzoïque ;
35. Acide 4-{2-[3-tert-butyl-4-(ethyl-*iso*-butyl-amino)-phenyl]-2-hydroxyimino-ethoxy}-2-hydroxy-benzoïque ;
36. Acide 4-[2-(3-tert-butyl-4-morpholin-4-yl-phenyl)-2-hydroxyimino-ethoxy]-2-hydroxy-benzoïque ;
37. Acide 4-[2-(3-tert-butyl-4-piperidin-1-yl-phenyl)-2-hydroxyimino-ethoxy]-2-hydroxy-benzoïque ;
38. Acide 4-{2-[3-tert-butyl-4-(2-oxo-piperidin-1-yl)-phenyl]-2-hydroxyimino-ethoxy}-benzoïque ;
39. Acide 4-{2-[3-tert-butyl-4-(2-oxo-pyrrolidin-1-yl)-phenyl]-2-hydroxyimino-ethoxy}-benzoïque ;
40. Acide 4-[2-(3-*tert*-butyl-4-ethylamino-phenyl)-2-hydroxyimino-ethoxy]-2-hydroxy-benzoïque ;
41. Acide 4-{2-[4-(ethyl-propionyl-amino)-3-*tert*-butyl-phenyl]-2-hydroxyimino-ethoxy}-2-hydroxy-benzoïque ;
42. Acide 4-{2-[3-tert-butyl-4-(ethoxycarbonyl-ethyl-amino)-phenyl]-2-hydroxyimino-ethoxy}-2-hydroxy-benzoïque ;
43. Acide 4-{2-[3-tert-butyl-4-(ethyl-methoxycarbonyl-amino)-phenyl]-2-hydroxyimino-ethoxy}-2-hydroxy-benzoïque ;
44. Acide 4-{2-[4-(ethyl-trifluoroacetyl-amino)-3-*tert*-butyl-phenyl]-2-hydroxyimino-ethoxy}-2-hydroxy-benzoïque ;
45. Acide 4-{2-[4-(trifluoroacetyl-amino)-3-*tert*-butyl-phenyl]-2-hydroxyimino-ethoxy}-2-hydroxy-benzoïque ;
46. Acide 4-{2-[3-tert-butyl-4-(ethyl-isopropyl-amino)-phenyl]-2-hydroxyimino-ethoxy}-2-hydroxy-benzoïque.

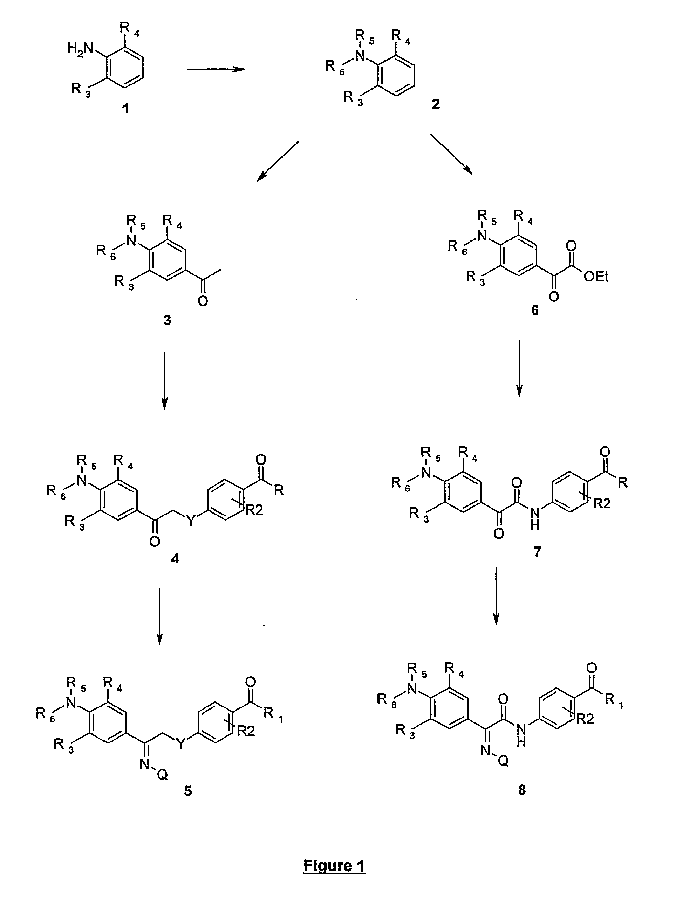

La présente invention a également pour objet les procédés de préparation des composés de formule (I), en particulier selon les schémas réactionnels donnés à la Figure 1.

Les composés revendiqués peuvent être obtenus à partir de produits de départ de type **1**. Après une étape de di-substitution de la fonction aniline (R₅=R₆) ou bien deux étapes de substitution de cette fonction, par exemple par acétylation (AcCl, Et₃N) puis alkylation (par exemple NaH, Alkyl-I), ou bien encore une unique étape de substitution (R₅=H), par exemple une alkylation (NaH, Alkyl-I), les composés de structure générale de type **2** sont obtenus.

Une acylation de type Friedel & Crafts permet d'obtenir les composés de structure **3** et **6**, respectivement par réaction de chlorure d'acétyle ou de chlorure d'éthyloxalyle en présence d'acide de Lewis comme AICl₃ par exemple.

Une α-Halogénation de **3**, suivie d'une substitution nucléophile avec la fonction chimique phénol, thiophénol ou aniline du partenaire correspondant au produit d'arrivée permet d'obtenir les composés de structure **4**, quand Y = O, S, N-R₁₇, respectivement.

Enfin, les composés de structure **5** peuvent être obtenus par réaction de la fonction cétone avec des dérivés de type Q-NH₂ dans l'éthanol, comme par exemple l'hydroxylamine (Q= OH). Dans le cas où R₁ = OH, une étape de saponification est ensuite nécessaire, par réaction de l'intermédiaire ester avec NaOH aqueux par exemple. Les composés de structure générale **7** peuvent être obtenus à partir des composés de type **6** par l'intermédiaire d'une saponification de la fonction ester suivie d'un couplage peptidique avec un dérivé aniline, comme le 4-amino benzoate d'éthyle par exemple. De même que pour la transition **4-5**, les composés de structure générale **8** peuvent être obtenus par réaction de la fonction cétone avec des dérivés de type Q-NH₂ dans l'éthanol, comme par exemple l'hydroxylamine (Q= OH). Dans le cas où R₁ = OH, une étape de saponification est ensuite nécessaire, par réaction de l'intermédiaire ester avec NaOH aqueux par exemple.

Les composés selon l'invention présentent des propriétés activatrices des récepteurs de type RARs. Cette activité activatrice des récepteurs RAR est mesurée dans un test de transactivation par la constante de dissociation Kdapp (apparent) et l'AC50 (concentration donnant 50% de l'activité de la molécule de référence).

Par activateur des récepteurs de type RARs, on entend selon l'invention tout composé qui pour au moins un des sous-types RARs présente une constante de dissociation Kdapp et une AC50, inférieures ou égales à 1 µM, dans un test de transactivation tel que décrit dans l'exemple 7.

Les composés préférés de la présente invention présentent pour au moins un des sous-types RARs, une constante de dissociation Kdapp inférieure ou égale à 500 nM, et avantageusement inférieure ou égale à 100 nM, et une AC50 ≤100 nM.

La présente invention a également pour objet les composés de formule (I) tels que décrits ci-dessus à titre de médicament.

Les composés selon l'invention conviennent particulièrement bien dans les domaines de traitement suivants :
1) pour traiter les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal) ;
3) pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanée telle que les kératoacanthomes ;
5) pour traiter d'autres désordres dermatologiques tels que les dermatoses immunes telles le lupus érythémateux, les maladies immunes bulleuses et les maladies du collagène, telle la sclérodermie ;
6) dans le traitement d'affections dermatologiques ou générales à composante immunologique ;
7) pour traiter certains troubles ophtalmologiques, notamment les cornéopathies,
8) pour prévenir ou guérir les stigmates de l'atrophie épidermique et/ou dermique induite par les corticostéroïdes locaux ou systémiques, ou toute autre forme d'atrophie cutanée,
9) dans le traitement de toute affection d'origine virale au niveau cutané ou général,
10) dans le traitement de désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose ;
11) pour lutter contre les troubles de la fonction sébacée tels que l'hyperséborrhée de l'acné ou la séborrhée simple ;
12) pour prévenir ou traiter les troubles de la cicatrisation, ou pour prévenir ou pour réparer les vergetures, ou encore pour favoriser la cicatrisation,
13) dans le traitement des désordres de la pigmentation, tel l'hyperpigmentation, le mélasma, l'hypopigmentation ou le vitiligo ;
14) dans le traitement des affections du métabolisme des lipides, tel l'obésité, l'hyperlipidémie, ou le diabète non insulino-dépendant ;
15) dans le traitement d'affections inflammatoires telles que l'arthrite ;
16) dans le traitement ou la prévention des états cancéreux ou précancéreux ;
17) dans la prévention ou le traitement de l'alopécie de différentes origines, notamment l'alopécie due à la chimiothérapie ou aux rayonnements ;
18) dans le traitement des troubles du système immunitaire, tel l'asthme, le diabète sucré de type I, la sclérose en plaque, ou autres disfonctionnements sélectifs du système immunitaire ; et
19) dans le traitement d'affections du système cardiovasculaire telles que l'artériosclérose ou l'hypertension.

La présente invention a également pour objet une composition pharmaceutique comprenant, dans un milieu physiologiquement acceptable, au moins un composé de formule (I) tel que défini ci-dessus.

La présente invention a aussi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou un de ses sels.

L'administration de la composition selon l'invention peut être effectuée par voie orale, entérale, parentérale, topique ou oculaire. De préférence, la composition pharmaceutique est conditionnée sous une forme convenant à une application par voie topique.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée. Par voie parentérale, la composition peut se présenter sous forme de solutions ou suspensions pour perfusion ou pour injection.

Les composés selon l'invention sont généralement administrés à une dose journalière d'environ 0,01 mg/kg à 100 mg/kg de poids corporel, en 1 à 3 prises.

Les composés sont utilisés par voie systémique à une concentration généralement comprise entre 0,001% et 10% en poids, de préférence entre 0,01% et 1% en poids, par rapport au poids de la composition.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée. Les composés sont utilisés par voie topique à une concentration généralement comprise entre 0,00 1 % et 10% en poids, de préférence entre 0,01% et 1% en poids, par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

L'invention a donc également pour objet une composition cosmétique comprenant, dans un support physiologiquement acceptable, au moins un des composés de formule (I).

L'invention a également pour objet l'utilisation non thérapeutique d'une composition cosmétique comprenant au moins un composé de formule (I) pour prévenir et/ou traiter les signes du vieillissement et/ou la peau sèche.

L'invention a aussi pour objet l'utilisation non thérapeutique d'une composition cosmétique comprenant au moins un composé de formule (I) pour l'hygiène corporelle ou capillaire.

La composition cosmétique selon l'invention contenant, dans un milieu physiologiquement acceptable, au moins un composé de formule (I) ou l'un de ses isomères optiques ou géométriques ou l'un de ses sels, peut se présenter notamment sous forme d'une crème, d'un lait, d'un gel, de suspensions de microsphères ou nanosphères ou vésicules lipidiques ou polymériques, de tampons imbibés, de solutions, de sprays, de mousses, de sticks, de savons, de bases lavantes ou de shampooings.

La concentration en composé de formule (I) dans la composition cosmétique est de préférence comprise entre 0,001% et 3% en poids, par rapport au poids total de la composition.

Par un milieu physiologiquement acceptable, on entend un milieu compatible avec la peau et éventuellement avec ses phanères (cils, ongles, cheveux) et/ou les muqueuses.

Les compositions pharmaceutiques et cosmétiques telles que décrites précédemment peuvent en outre contenir des additifs inertes, ou même pharmacodynamiquement actifs pour ce qui concerne les compositions pharmaceutiques, ou des combinaisons de ces additifs, et notamment :
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique ;
- des agents émulsionnants ;
- des filtres UV-A et UV-B ;
- des antioxydants, tels que l'α-tocophérol, le butylhydroxyanisole ou le butylhydroxytoluène, la Super Oxyde Dismutase, l'Ubiquinol ou certains chélatants de métaux ;
- des agents dépigmentants tels que l'hydroquinone, l'acide azélaïque, l'acide caféïque ou l'acide kojique ;
- des émollients ;
- des agents hydratants comme le glycérol, le PEG 400, la thiamorpholinone, et ses dérivés ou l'urée ;
- des agents antiséborrhéiques ou antiacnéiques, tels que la S-carboxyméthylcystéine, la S-benzyl-cystéamine, leurs sels ou leurs dérivés, ou le peroxyde de benzoyle ;
- des antibiotiques comme l'érythromycine et ses esters, la néomycine, la clindamycine et ses esters, les tétracyclines ;
- des agents antifongiques tels que le kétoconazole ou les polyméthylène-4,5 isothiazolidones-3 ;
- des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïne (5,4-diphényl-imidazolidine 2,4-dione) ;
- des agents anti-inflammatoires non stéroïdiens ;
- des caroténoïdes et, notamment, le β-carotène ;
- des agents anti-psoriatiques tels que l'anthraline et ses dérivés;
- des acides eicosa-5,8,11,14-tétraynoïque et eicosa-5,8,11-triynoïque, leurs esters et amides ;
- des rétinoïdes, c'est à dire des ligands des récepteurs RXR, naturels ou synthétiques ;
- des corticostéroïdes ou des oestrogènes ;
- des α-hydroxy acides et des α-céto acides ou leurs dérivés, tels que les acides lactique, malique, citrique, glycolique, mandélique, tartrique, glycérique, ascorbique, ainsi que leurs sels, amides ou esters, ou des β-hydroxy acides ou leurs dérivés, tels que l'acide salicylique ainsi que ses sels, amides ou esters ;
- des bloqueurs de canaux ioniques tels que les canaux potassiques ;
- ou encore, plus particulièrement pour les compositions pharmaceutiques, en association avec des médicaments connus pour interférer avec le système immunitaire (par exemple, la cyclosporine, le FK 506, les glucocorticoïdes, les anticorps monoclonaux, les cytokines ou les facteurs de croissance...).

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

Un autre objet de l'invention se rapporte à un procédé cosmétique d'embellissement de la peau, caractérisé par le fait que l'on applique sur la peau une composition comprenant au moins un composé de formule (I) tel que défini précédemment.

L'activation des récepteurs de l'acide rétinoique par les composés de formule (I) suivant l'invention permet d'obtenir une peau dont l'aspect de surface est embelli.

On va maintenant donner, à titre d'illustration , plusieurs exemples d'obtention de composés actifs de formule (I) selon l'invention, des résultats d'activité biologique ainsi que diverses formulations concrètes à base de tels composés.

### EXEMPLES

### Exemple 1 : Acide 4-[2-(3-tert-butyl-4-diethylamino-phenyl)-2-hydroxyimino-ethoxy]-benzoïque

### a. 4-Bromo-2-tert-butylaniline

51,8 g (347 mmol) de 2-tert-butyl-aniline (produit commercial vendu notamment par la société Aldrich) sont dissous dans 600 ml d'acide acétique glacial. 900 ml d'acide hydrobromique aqueux 48% sont alors ajoutés, puis le milieu réactionnel est refroidi à 0°C. 300 ml de DMSO sont alors ajoutés goutte à goutte, puis le milieu réactionnel est ramené à température ambiante. Après 4 heures d'agitation, 250 ml d'acétate d'éthyle sont ajoutés, suivis de 400 ml de soude 5N, puis 1,75 l de soude 10N, pour ramener le pH à 8. 250 ml d'acétate d'éthyle sont ajoutés, et le milieu est décanté. La phase aqueuse est ensuite ré-extraite avec 500 ml d'acétate d'éthyle. Les phases organiques combinées sont alors rincées avec 1 l d'eau, puis concentrées sous pression réduite. Le résidu obtenu est purifié par chromatographie (éluant heptane 95 / acétate d'éthyle 5). Une huile orangée est obtenue (m = 56.2 g, Rendement = 71 %)

### b. (4-Bromo-2-tert butyl-phenyl)-diethyl-amine

20 g (88 mmol) de 4-bromo-2-*tert*-butylaniline sont dissous dans 200 ml de DMSO sous atmosphère d'azote. 7,7 g (190 mmol) d'hydrure de sodium sont ajoutés par portions. Après 30 minutes d'agitation, 15,4 ml (190 mmol) d'iodure d'éthyle sont ajoutés goutte à goutte. Le milieu réactionnel est agité pendant 12 heures, puis versée sur une solution saturée de chlorure d'ammonium. Après extraction avec de l'acétate d'éthyle, la séquence réactionnelle est répétée, puis le résidu est purifié par chromatographie sur colonne de silice (éluant Heptane 98 / acétate d'éthyle 2). Une huile incolore est obtenue (m = 16,7 g, Rendement = 67%).

### c. 3-tert-Butyl-4-diethylamino-benzaldehyde

16,7 g (58 mmol) de (4-bromo-2-*tert*-butyl-phenyl)-diethyl-amine sont dissous dans 250 ml de THF anhydre, et le mélange est refroidi à -78°C. 35 ml (88 mmol) d'une solution de butyllithium 2,5 M sont ajoutés goutte à goutte, puis le milieu est agité pendant 30 minutes. 6,9 ml (88 mmol) de diméthylformamide sont additionnés goutte à goutte, puis le milieu réactionnel est lentement ramené à température ambiante. Après 1 heure d'agitation, le milieu réactionnel est traité par une solution saturée de chlorure d'ammonium, et extrait avec de l'acétate d'éthyle. Le résidu obtenu est purifié par chromatographie sur colonne de silice. Une huile jaune est obtenue (m = 11,5 g, Rendement = 85%).

### d. 3-tert-Butyl-4-diethylamino-acétophenone

11,5 g (49 mmol) de 3-*tert* butyl-4-diethylamino-benzaldehyde sont dissous dans 200 ml de THF. Une solution 3 M de bromure de méthylmagnesium (21 ml, 63 mmol) est additionnée puis le milieu est agité pendant 2 heures. Après traitement par une solution saturée de chlorure d'ammonium puis extraction avec de l'acétate d'éthyle, le résidu brut obtenu est dissous dans 200 ml de dichlorométhane, et 10 g (120 mmol) de dioxyde de manganese sont ajoutés. Le milieu réactionnel est agité pendant 24 heures, puis 10 g de MnO2 sont de nouveau ajoutés. Après 12 heures d'agitation, le milieu réactionnel est filtré, et le filtrat concentré sous pression réduite. Le résidu obtenu est purifié par chromatographie sur colonne de silice (éluant heptane 9/ acétate d'éthyle 10). Une huile jaune est obtenue (m = 5,6 g, Rendement = 46%).

### e. 2-Bromo-1-(3-tert-butyl-4-diethylamino-phenyl)-ethanone

2,1 g (8 mmol) de 3-*tert*-butyl-4-diethylamino-acétophenone sont dissous dans 250ml d'éther éthylique et 25 ml de dioxanne. Une solution de dibrome (0,43 ml dans 50 ml de dichlorométhane, 8 mmol) est rajoutée goutte à goutte pendant 1 h 30 à 0°C. La solution obtenue est alors versée sur de l'eau glacée et la phase organique est lavée par une solution saturée de thiosulfate de sodium. Le résidu obtenu après concentration est purifié par chromatographie (éluant heptane 99 / acétate d'éthyle 1). Une huile jaune est obtenue (m = 2g, Rendement = 73%).

### f. 4-[2-(3-tert-Butyl-4-diethylamino-phenyl)-2-oxo-ethoxy]-benzoate de méthyle

1 g (3 mmol) de 2-bromo-1-(3-*tert*-butyl-4-diethylamino-phenyl)-ethanone et 444 mg (3 mmol) de 4-hydroxybenzoate de méthyle sont dissous dans 50 ml de 2-butanone. 440 mg (3 mmol) de carbonate de potassium et une quantité catalytique de 18-crown-6 sont additionnés. Le milieu réactionnel est chauffé à reflux pendant 12 heures, puis filtré et concentré sous pression réduite. Le résidu obtenu est purifié par chromatographie (éluant Heptane 9 / acétate d'éthyle 1). Un solide blanc est obtenu (pf = 92°C, m = 620 mg, Rendement = 54%).

### g. 4-[2-(3-tert-Butyl-4-diethylamino-phenyl)-2-hydroxyimino-ethoxy]-benzoate de méthyle

620 mg (1,6 mmol) de 4-[2-(3-*tert*-butyl-4-diethylamino-phenyl)-2-oxo-ethoxy]-benzoate de méthyle sont dissous dans 30 ml de THF et 30 ml de méthanol. 0,63 ml (7,8 mmol) de pyridine et 540 mg (7,8 mmol) de chlorhydrate d'hydroxylamine sont ajoutés. Le milieu réactionnel est chauffé à reflux pendant 1 h30 puis, après refroidissement, est versé dans une solution saturée de chlorure d'ammonium. Après extraction avec de l'acétate d'éthyle et concentration, le résidu est purifié par chromatographie (éluant Heptane 9 / acétate d'éthyle 1). Les deux isomères sont isolés séparément : l'isomère syn est le produit majoritaire (m = 550 mg, Rendement = 83%), l'isomère anti est minoritaire (m = 90 mg, Rendement = 13%).

### h. Acide 4-[2-(3-tert-butyl-4-diethylamino-phenyl)-2-hydroxyimino-ethoxy]-benzoïque

550 mg (1,4 mmol) de 4-[2-(3-*tert*-butyl-4-diethylamino-phenyl)-2-hydroxyimino-ethoxy]-benzoate de méthyle sont dissous dans 10 ml de THF. 1 ml de méthanol est ajouté, suivi de 164 mg (4 mmol) d'hydroxyde de sodium en poudre et 100 µL d'eau. Le milieu réactionnel est agité à température ambiante pendant 12 heures, puis traité par une solution saturée de chlorure d'ammonium. La phase aqueuse est ramenée à pH 5 par ajout d'acide chlorhydrique 1 N, et deux extractions avec de l'acétate d'éthyle sont réalisées. Le résidu obtenu est purifié par chromatographie sur colonne de silice (éluant Heptane 80 / heptane 20 puis 50/50). Le produit final est ensuite recristallisé dans un système heptane / acétone. Un solide cristallin blanc est obtenu (pf = 185°C, m = 245 mg, Rendement = 44%) ; (RMN ¹H (CDCl₃): 1,04-1,07 (t, 6H); 1,45 (s, 9H); 2,89 (m, 4H); 5,36 (s, 2H); 7,00-7,02 (d, J=8,8 Hz, 2H); 7,22- 7,25 (d, J=8,3 Hz, 1 H); 7,44- 7,46 (dd, J= 6,2 Hz, J'= 2,06 Hz, 1H); 7,72 (d, J=2,0 Hz, 1H); 8,03- 8,05 (d, J= 8,74 Hz, 2H)).

### Exemple 2. Acide 4-[2-(3-tert-butyl-4-diethylamino-phenyl)-2-hydroxyimino-ethoxy]-2-hydroxy-benzoïque

### a. 4-[2-(3-tert-Butyl-4-diethylamino-phenyl)-2-oxo-ethoxy]-2-hydroxy-benzoate de méthyle

De manière analogue à l'exemple 1.f. par réaction de 800 mg (2,5 mmol) de 2-bromo-1-(3-*tert*-butyl-4-diethylamino-phenyl)-ethanone (exemple 1.e) et 386 mg (2,3 mmol) de 2,4-dihydroxybenzoate de méthyle en présence de 350 mg (2,5 mmol) de carbonate de potassium et une quantité catalytique de 18-crown-6. Une huile brune est obtenue (m = 600 mg, Rendement = 63%).

### b. 4-[2-(3-tert-Butyl-4-diethylamino-phenyl)-2-hydroxyimino-ethoxy]-2-hydroxybenzoate de méthyle

De manière analogue à l'exemple 1.g. par réaction de 300 mg (0,7 mmol) de 4-[2-(3-*tert-*butyl-4-diethylamino-phenyl)-2-oxo-ethoxy]-2-hydroxy-benzoate de méthyle en présence de 0, 3 ml (3,6 mmol) de pyridine et 250 mg (3,6 mmol) de chlorhydrate d'hydroxylamine. Les deux isomères sont isolés séparément : l'isomère syn est le produit majoritaire (m = 120 mg, Rendement = 39%), l'isomère anti est minoritaire (m = 50 mg, Rendement = 16%).

### c. Acide 4-[2-(3-tert-butyl-4-diethylamino-phenyl)-2-hydroxyimino-ethoxy]-2-hydroxy-benzoïque

De manière analogue à l'exemple 1.g. par réaction de 120 mg (0,3 mmol) de 4-[2-(3-*tert-*butyl-4-diethylamino-phenyl)-2-hydroxyimino-ethoxy]-2-hydroxy-benzoate de méthyle avec 46 mg (1,4 mmol) d'hydroxyde de sodium en poudre. Le produit final est ensuite recristallisé dans un système heptane / éther éthylique. Un solide cristallin blanc est obtenu (pf = 210°C, m = 60 mg, Rendement = 48%); (RMN ¹H(DMSO): 0,96-1,00 (t, 6H) ; 1,38 (s, 9H) ; 2,7-3 (m, 4H) ; 5,26 (s, 2H) ; 6,46- 6,49 (dd, J=6,4 Hz, J'= 2,4 Hz, 1 H) ; 6,55 (d, J=2,4 Hz, 1H) ; 7,26- 7,28 (d, J= 8,3 Hz, 1H) ; 7,46- 7,48 (dd, J= 6,2 Hz, J'= 2,03 Hz, 1 H) ; 7,65- 7,67 (d, J= 8,8 Hz, 1 H) ; 7,69 (d, J= 2,03 Hz, 1 H) ; 11,9 (s, 1 H) ; 13-14 (s, 1H)).

### Exemple 3. Acide 4-[2-(3-tert-butyl-4-pyrrolidin-1-yl-phenyl)-2-hydroxyimino-ethoxy]-benzoïque

### a. 1-(4-Bromo-2-tert-butyl-phenyl)-pyrrolidine

De manière analogue à l'exemple 1.b, par réaction de 20 g (88 mmol) de 4-bromo-2-*tert-*butylaniline avec 7,6 g (194 mmol) d'hydrure de sodium et 22,2 ml (194 mmol) de dibromobutane. Une huile jaune est obtenue (m = 18g, Rendement = 73%).

### b. 3-tert-Butyl-4-pyrrolidin-1-yl-benzaldehyde

De manière analogue à l'exemple 1.c, par réaction de 10 g (35 mmol) de 1-(4-bromo-2-*tert*-butyl-phenyl)-pyrrolidine avec 21 ml de butyllithium 2,5 M et 4 ml (53 mmol) de diméthylformamide. Une huile jaune est obtenue (m = 4,3 g, Rendement = 53%).

### c. 3-tert-Butyl-4-pyrrolidin-1-yl-acetophenone

De manière analogue à l'exemple 1.d, par réaction de 4,3 g (19 mmol) de 3-*tert*-butyl-4-pyrrolidin-1-yl-benzaldehyde avec 8,2 ml d'une solution 3M de bromure de méthylmagnesium, puis avec 15,3 g (180 mmol) de dioxyde de manganèse. Une huile orangée est obtenue (m = 2,3 g, Rendement = 52%).

### d. 2-Bromo-1-(3-tert-butyl-4-pyrrolidin-1-yl-phenyl)-ethanone

De manière analogue à l'exemple 1.e, par réaction de 2,3 g (9,4 mmol) de 3-*tert*-butyl-4-pyrrolidin-1-yl-acetophenone avec 240 µL de dibrome. Une huile jaune est obtenue (m = 2,3 g, Rendement = 76%).

### e. 4-[2-(3-tert-Butyl-4-pyrrolidin-1-yl-phenyl)-2-oxo-ethoxy]-benzoate de méthyle

De manière analogue à l'exemple 1.f, par réaction de 600 mg (1,9 mmol) de 2-bromo-1-(3-*tert*-butyl-4-pyrrolidin-1-yl-phenyl)-ethanone et 268 mg (1,8 mmol) de 4-hydroxybenzoate de méthyle, puis 248 mg (2 mmol) de carbonate de potassium et une quantité catalytique de 18-crown-6. Une huile orangée est obtenue (m = 400 mg, Rendement = 53%).

### f. 4-[2-(3-tert-Butyl-4-pyrrolidin-1-yl-phenyl)-2-hydroxyimino-ethoxy]-benzoate de méthyle

De manière analogue à l'exemple 1.g, par réaction de 400 mg (1 mmol) de 4-[2-(3-*tert-*butyl-4-pyrrolidin-1-yl-phenyl)-2-oxo-ethoxy]-benzoate de méthyle avec 0,4 ml (5 mmol) de pyridine et 352 mg (5 mmol) de chlorhydrate d'hydroxylamine. Un solide rosâtre est obtenu (pf = 170°C, m = 210 mg, Rendement = 51 %).

### g. Acide 4-[2-(3-tert-Butyl-4-pyrrolidin-1-yl-phenyl)-2-hydroxyimino-ethoxy]-benzoïque

De manière analogue à l'exemple 1.h, par réaction de 210 mg (0,5 mmol) de 4-[2-(3-*tert-*butyl-4-pyrrolidin-1-yl-phenyl)-2-hydroxyimino-ethoxy]-benzoate de méthyle avec 100 mg (2,5 mmol) d'hydroxyde de sodium. Un solide blanc est obtenu après recristallisation (pf = 235°C, m = 44 mg, Rendement = 22%) ; (RMN ¹H (DMSO): 1,35 (s, 9H) ; 1,86 (s, 4H) ; 2,89 (s, 4H) ; 5,29 (s, 2H); 7,04- 7,06 (d, J= 7,65 Hz, 2H) ; 7,40- 7,42 (d, J= 7,4 Hz, 1H) ; 7,49- 7,51 (d, J= 7,4 Hz, 1 H) ; 7,65 (s, 1 H) ; 7,86- 7,88 (d, J= 7,4 Hz, 2H) ; 11,9 (s, 1H) ; 12,6 (s, 1H)).

### Exemple 4: Acide 4-[2-(3-tert-butyl-4-pyrrolidin-1-yl-phenyl)-2-hydroxyimino ethoxy]-2-hydroxy-benzoïque

### a. 4-[2-(3-tert-Butyl-4-pyrrolidin-1-yl-phenyl)-2-oxo-ethoxy]-2-hydroxy-benzoate de méthyle

De manière analogue à l'exemple 1.f. par réaction de 600 mg (1,9 mmol) de 2-bromo-1-(3-*tert*-butyl-4-pyrrolidin-1-yl-phenyl)-ethanone (exemple 1.e) et 302 mg (1,8 mmol) de 2,4-dihydroxybenzoate de méthyle en présence de 250 mg (2 mmol) de carbonate de potassium et une quantité catalytique de 18-crown-6. Une huile brune est obtenue (m = 350 mg, Rendement = 47%).

### b. 4-[2-(3-tert-Butyl-4- pyrrolidin-1-yl-phenyl)-2-hydroxyimino-ethoxy]-2-hydroxybenzoate de méthyle

De manière analogue à l'exemple 1.g. par réaction de 350 mg (0,85 mmol) de 4-[2-(3-*tert*-butyl-4-pyrrolidin-1-yl-phenyl)-2-oxo-ethoxy]-2-hydroxy-benzoate de méthyle en présence de 0, 35 ml (4,3 mmol) de pyridine et 300 mg (4,3 mmol) de chlorhydrate d'hydroxylamine. Les deux isomères sont isolés séparément : l'isomère syn est le produit majoritaire, sous forme de solide rosâtre (pf = 149°C, m = 193 mg, Rendement = 53%).

### c. Acide 4-[2-(3-tert-butyl-4- pyrrolidin-1-yl-phenyl)-2-hydroxyimino-ethoxy]-2-hydroxy-benzoïque

De manière analogue à l'exemple 1.g. par réaction de 193 mg (0,45 mmol) de 4-[2-(3-*tert*-butyl-4- pyrrolidin-1-yl-phenyl)-2-hydroxyimino-ethoxy]-2-hydroxy-benzoate de méthyle avec 90 mg (2,3 mmol) d'hydroxyde de sodium en poudre. Le produit final est ensuite recristallisé dans un système heptane / éther éthylique. Un solide cristallin beige est obtenu (pf = 234°C, m = 25 mg, Rendement = 13%) ; (RMN ¹H (DMSO): 1,35 (s, 9H); 1,86 (s, 4H); 2,89 (s, 4H); 5,26 (s, 2H); 6,46- 6,49 (d, J= 8,6 Hz, 1 H); 6,55 (s, 1 H); 7,40-7,42 (d, J= 8,2 Hz, 1H); 7,49- 7,51 (d, J= 7,9 Hz, 1H); 7,66- 7,68 (d, J= 8,5 Hz, 2H); 11,9 (s, 1H)).

### Exemple 5 : Acide 4-{2-[4-(acetyl-ethyl-amino)-3-tert-butyl-phenyl]-2-hydroxyimino-ethoxy}-benzoïque

### a. N-(2-tert-Butyl-phenyl)-acetamide

40 g (268 mmol) de 2-*tert*-butylaniline (produit commercial vendu notamment par la société Aldrich) sont dissous dans 700 ml de THF. 32,5 g (320 mmol) de triethylamine sont ajoutés, et le milieu est refroidi à 5°C. 21 ml (295 mmol) de chlorure d'acétyle sont additionnés et la réaction est agitée pendant 2 heures puis versée dans de l'eau. Après extraction avec de l'acétate d'éthyle et rinçage à l'eau, puis concentration, un solide cotonneux est obtenu (pf = 163°C, m = 49,4 g, Rendement = 97%).

### b. N-(4-Bromo-2-tert-butyl-phenyl)-acetamide

45 g (235 mmol) de N-(2-*tert*-butyl-phenyl)-acetamide sont dissous dans 450 ml de dichloromethane. 27,7 g (235 mmol) de N-bromosuccinimide sont alors ajoutés, et le milieu réactionnel est agité pendant 48 heures, puis filtré. Le résidu obtenu après concentration du filtrat est dissous dans un mélange acétate d'éthyle / eau, et la phase organique est lavée à l'eau. Le résidu obtenu est remis en réaction en présence de 15 g de N-bromosuccinimide, puis traité de la même manière après 48 heures. Le résidu finalement obtenu est repris dans de l'acétate d'éthyle, rincé avec une solution de thiosulfate de sodium puis par de l'eau et concentré sous pression réduite. Un solide orange pâle est obtenu (m = 63 g, Rendement = 100%).

### c. N-(4-Bromo-2-tert-butyl-phenyl)-N-ethyl-acetamide

2,7 g (10 mmol) de N-(4-bromo-2-*tert* butyl-phenyl)-acetamide sont dissous dans 30 ml de DMSO. 420 mg (10,5 mmol) d'hydrure de sodium sont ajoutés par portions, et le milieu est agité pendant 20 minutes à température ambiante. 1,13 ml (11 mmol) d'iodure d'éthyle sont alors ajoutés, et le milieu réactionnel est agité pendant 14 heures. Après traitement par une solution de chlorure d'ammonium et extraction avec de l'acétate d'éthyle, le résidu obtenu est purifié par chromatographie (éluant heptane 9/ acétate d'éthyle 1, puis 75/25). Une huile orange épaisse est obtenue (m = 2,45 g, Rendement = 82%).

### d. N-(4-Acetyl-2-tert-butyl-phenyl)-N-ethyl-acetamide

2 g (6,7 mmol) de N-(4-bromo-2-*tert*-butyl-phenyl)-N-ethyl-acetamide, 1,34 g (13,4 mmol) de butylvinyl ether, 3,73 ml (26,8 mmol) de triethylamine, 60 mg (0,27 mmol) d'acétate de palladium, 122 mg (0,4 mmol) de tri-o-tolylphosphine sont dissous dans 20 ml d'acétonitrile. Le milieu réactionnel est chauffé à reflux pendant 6 heures, puis laissé à température ambiante pendant 12 heures. Après traitement par une solution d'acide chlorhydrique 1 N et extraction avec de l'acétate d'éthyle, le résidu obtenu est purifié par chromatographie (éluant heptane 90 / acétate d'éthyle 1). Une huile orange est obtenue (m = 620 mg, Rendement = 35%).

### e. N-[4-(2-Bromo-acetyl)-2-tert-butyl-phenyl]-N-ethyl-acetamide

600 mg (2,3 mmol) de N-(4-acetyl-2-*tert*-butyl-phenyl)-N-ethyl-acetamide sont dissous dans 20 ml de THF, et le milieu réactionnel est refroidi à 0°C. 860 mg (2,3 mmol) de tribromure de phenyltrimethylammonium sont ajoutés, et le milieu réactionnel est agité à température ambiante pendant 14 heures. Après traitement par une solution d'acide chlorhydrique 1 N et extraction avec de l'acétate d'éthyle, le résidu obtenu est purifié par chromatographie. Une huile jaune est obtenue (m = 600 mg, Rendement = 77%).

### f. 4-{2-[4-(Acetyl-ethyl-amino)-3-tert-butyl-phenyl]-2-oxo-ethoxy}-benzoate d'allyle

De manière analogue à l'exemple 1.f, par réaction de 300 mg (0,9 mmol) de N-[4-(2-bromo-acetyl)-2-*tert*-butyl-phenyl]-N-ethyl-acetamide avec 157 mg (0,9 mmol) de 4-hydroxybenzoate d'allyle et 134 mg (0.97 mmol) de carbonate de potassium.Une huile orange est obtenue (m = 360 mg, Rendement = 94%).

### g. 4-{2-[4-(Acetyl-ethyl-amino)-3-tert-butyl-phenyl]-2-hydroxyimino-ethoxy}-benzoate d'allyle

De manière analogue à l'exemple 1.g, par réaction de 360 mg (0.8 mmol) de 4-{2-[4-(acetyl-ethyl-amino)-3-*tert*-butyl-phenyl]-2-oxo-ethoxy}-benzoate d'allyle avec 284 mg (4 mmol) de chlorhydrate d'hydroxylamine et 320µL de pyridine. Une pâte jaunâtre est obtenue (m = 360 mg, Rendement = 96%).

### h. Acide 4-{2-[4-(acetyl-ethyl-amino)-3-tert-butyl-phenyl]-2-oxo-ethoxy}-benzoïque

360 mg (0,8 mmol) de 4-{2-[4-(acetyl-ethyl-amino)-3-tert-butyl-phenyl]-2-hydroxyimino-ethoxy}-benzoate d'allyle sont dissous dans 10 ml de THF. 48 mg (0,04 mmol) de tetrakis-triphenylphosphinopalladium sont additionnés, et la milieu est agité pendant 5 minutes. 73µl (0,8 mmol) de morpholine sont alors additionnés, et le milieu réactionnel est agité pendant 15 heures. Après traitement par une solution d'acide chlorhydrique 1 N et extraction avec de l'acétate d'éthyle, le résidu est purifié par chromatographie (éluant heptane 5/ acétate d'éthyle 5). Une poudre blanche est obtenue (pf = 192°C, m = 200 mg, Rendement = 60%); (RMN ¹H (DMSO): 1,05- 1,07 (t, 3H); 1,29 (s, 9H); 1,64 (s, 3H); 2,73 (m, 1H); 4,13 (m, 1H); 5,33 (s, 2H); 6,46 (1 H); 6,6 (1 H); 7,07- 7,09 (d, J= 8,1 Hz, 1 H); 7,51- 7,53 (d, J= 8 Hz, 1 H); 7,54- 7,64 (m, 2H); 7,86 (s, 1 H); 12,1 (s, 1H)).

### Exemple 6 : Acide 4-{2-[4-(acetyl-ethyl-amino)-3-tert-butyl-phenyl]-2-hydroxyimino-ethoxy}-2-hydroxy-benzoïque

### a. 4-{2-[4-(Acetyl-ethyl-amino)-3-tert-butyl-phenyl]-2-oxo-ethoxy}-2-hydroxybenzoate d'allyle

De manière analogue à l'exemple 1.f, par réaction de 300 mg (0,9 mmol) de N-[4-(2-bromo-acetyl)-2-*tert*-butyl-phenyl]-N-ethyl-acetamide avec 157 mg (0,9 mmol) de 2,4-dihydroxybenzoate d'allyle et 134 mg (0.97 mmol) de carbonate de potassium. Une huile orange est obtenue (m = 370 mg, Rendement = 93%).

### b. 4-{2-[4-(Acetyl-ethyl-amino)-3-tert-butyl-phenyl]-2-hydroxyimino-ethoxy}-2-hydroxy-benzoate d'allyle

De manière analogue à l'exemple 1.g, par réaction de 370 mg (0,8 mmol) de 4-{2-[4-(acetyl-ethyl-amino)-3-*tert*-butyl-phenyl]-2-oxo-ethoxy}-2-hydroxy-benzoate d'allyle avec 284 mg (4 mmol) de chlorhydrate d'hydroxylamine et 320µL de pyridine. Une pâte jaunâtre est obtenue (m = 380 mg, Rendement = 99%).

### c. Acide 4-{2-[4-(acetyl-ethyl-amino)-3-tert-butyl-phenyl]-2-oxo-ethoxy}-2-hydroxy-benzoïque

De manière analogue à l'exemple 5.h, par réaction de 380 mg (0,8 mmol) de 4-{2-[4-(acetyl-ethyl-amino)-3-tert-butyl-phenyl]-2-hydroxyimino-ethoxy}-2-hydroxy-benzoate d'allyle avec 48 mg (0,04 mmol) de tetrakis-triphenylphosphinopalladium et 73µL (0,8 mmol) de morpholine. Une poudre blanche est obtenue (pf = 199°C, m = 200 mg, Rendement = 59%) ; (RMN ¹H (DMSO): 1,05- 1,08 (t, 3H); 1,29 (s, 9H); 1,64 (s, 3H); 2,7-2,8 (m, 1 H); 4,1- 4,2 (m, 1 H); 5,34 (s, 2H); 6,44- 6,47 (dd, J= 6,8 Hz, J'= 2,2 Hz, 1 H); 6,53 (d, J= 2 Hz, 1 H); 7,07- 7,09 (d, J= 8,2 Hz, 1 H); 7,51- 7,53 (dd, J= 6,3 Hz, J'= 1,86 Hz, 1 H); 7,65- 7,67 (d, J= 8,8 Hz, 1 H); 7,86 (d, J= 1,78 Hz, 1 H); 12,2 (s, 1H)).

### EXEMPLE 7 : TEST DE TRANSACTIVATION

L'activation des récepteurs par un agoniste (activateur) dans des cellules HeLa conduit à l'expression d'un gène reporter, la luciférase, qui, en présence d'un substrat génère de la lumière. On peut donc mesurer l'activation des récepteurs en quantifiant la luminescence produite après incubation des cellules en présence d'un antagoniste de référence. Les produits activateurs vont déplacer l'antagoniste de son site permettant ainsi l'activation du récepteur. La mesure de l'activité se fait par la quantification de la hausse de la lumière produite. Cette mesure permet de déterminer l'activité activatrice des composés selon l'invention.

Dans cette étude, nous déterminons une constante qui représente l'affinité de la molécule pour le récepteur. Cette valeur pouvant fluctuer selon l'activité basale et l'expression du récepteur, on la dénomine Kd apparent (KdApp).

Pour déterminer cette constante, des « courbes croisées » du produit à tester contre un antagoniste de référence, l'acide 4-(5,5-Dimethyl-8-p-tolyl-5,6-dihydro-naphthalen-2-ylethynyl)-benzoïque sont réalisées en plaque 96 puits. Le produit à tester est utilisé à 10 concentrations et l'antagoniste de référence à 7 concentrations. Dans chaque puit, les cellules sont en contact d'une concentration du produit à tester et d'une concentration de l'antagoniste de référence, l'acide 4-(5,5-Dimethyl-8-p-tolyl-5,6-dihydro-naphthalen-2-ylethynyl)-benzoique. Des mesures sont également réalisées pour les témoins agoniste total (l'acide 4-[2-(5,5,8,8 tetramethyl-5,6,7,8 tetrahydronaphtalene-2-yl)propenyl]-benzoique) et agoniste inverse, l'acide 4-{(E)-3-[4-(4-tert-Butyl-phenyl)-5,5,8,8-tetramethyl-5,6,7,8-tetrahydro-naphthalen-2-yl]-3-oxo-propenyl}-benzoïque.

Ces courbes croisées nous permettent de déterminer les AC50 (concentration à laquelle on observe 50% d'activation) du ligand de référence à différentes concentrations de produit à tester. Ces AC50 sont utilisées pour calculer la régression de Schild en traçant une droite répondant à l'équation de Schild (« quantitation in receptor pharmacology » Terry P.Kenakin, Receptors and Channels, 2001,7, 371-385).

Dans le cas d'un agoniste, nous calculons une AC50 (concentration donnant 50% de l'activité) en traçant la courbe du produit à la concentration du ligand de référence donnant 80% d'activation.

Les lignées cellulaires HeLa utilisées sont des transfectants stables contenant les plasmides ERE-βGlob-Luc-SV-Neo (gène reporter) et RAR (α, β, γ) ER-DBD-puro. Ces cellules sont ensemencées en plaques 96 puits à raison de 10 000 cellules par puit dans 100µl de milieu DMEM sans rouge de phénol et supplémenté par 10% de sérum de veau délipidé. Les plaques sont ensuite incubées à 37°C, 7% CO₂ pour 4 H.

Les différentes dilutions des produits à tester, du ligand de référence (l'acide 4-(5,5-Dimethyl-8-p-tolyl-5,6-dihydro-naphthalen-2-ylethynyl)-benzoique), du témoin 100% (l'acide 4-[2-(5,5,8,8 tetramethyl-5,6,7,8 tetrahydronaphtalene-2-yl)propenyl]-benzoïque 100 nM) et du témoin 0% (l'acide 4-{(E)-3-[4-(4-tert-Butyl-phenyl)-5,5,8,8-tetramethyl-5,6,7,8-tetrahydro-naphthalen-2-yl]-3-oxo-propenyl}-benzoïque 500 nM) sont rajoutées à raison de 5 µl par puits. Les plaques sont ensuite incubées 18 heures à 37°C, 7% CO₂.

Le milieu de culture est éliminé par retournement et 100 µl d'un mélange 1 :1 PBS/Luciferine est ajouté à chaque puit. Après 5 minutes, les plaques sont lues par le lecteur de luminescence.

| | RaRalpha | | RARbeta | | RARgamma | |
|---|---|---|---|---|---|---|
| | **Kd app (nM)** | **AC50 (nM)** | **Kd app (nM)** | **AC50(nM)** | **Kd app (nM)** | **AC50(nM)** |
| Ex1 | NA | NA | 500 | 2500 | 60 | 80 |
| Ex2 | 5000 | 5000 | 500 | 1000 | 4 | 4 |

Les résultats obtenus avec les composés selon l'invention montrent bien des Kdapp ≤ 100 nM et une AC50 ≤ 100 nM pour au moins un des sous-types de récepteur ceci démontrant bien une augmentation du signal, de la luminescence en présence de l'antagoniste de référence. Les composés selon l'invention sont donc bien activateurs des récepteurs de l'acide rétinoique (RAR).

### EXEMPLE 8 : EXEMPLES DE FORMULATION

Dans cet exemple, on a illustré diverses formulations concrètes à base des composés selon l'invention.

| A- VOIE ORALE | | |
|---|---|---|
| | (a) Comprimé de 0,2 g | |
| | - Composé de l'exemple 6 | 0,001 g |
| | - Amidon | 0,114 g |
| | - Phosphate bicalcique | 0,020 g |
| | - Silice | 0,020 g |
| | - Lactose | 0,030 g |
| | - Talc | 0,010 g |
| | - Stéarate de magnésium | 0,005 g |
| | (b) Suspension buvable en ampoules de 5 ml | |
| | - Composé de l'exemple 3 | 0,001 g |
| | - Glycérine | 0,500 g |
| | - Sorbitol à 70% | 0,500 g |
| | - Saccharinate de sodium | 0,010 g |
| | - Parahydroxybenzoate de méthyle | 0,040 g |
| | - Arome | qs |
| | - Eau purifiée | qsp 5 ml |
| | (c) Comprimé de 0,8 g | |
| | - Composé de l'exemple 4 | 0,500 g |
| | - Amidon prégélatinisé | 0,100 g |
| | - Cellulose microcristalline | 0,115 g |
| | - Lactose | 0,075 g |
| | - Stéarate de magnésium | 0,010 g |
| | (d) Suspension buvable en ampoules de 10 ml | |
| | - Composé de l'exemple 2 | 0,200 g |
| | - Glycérine | 1,000 g |
| | - Sorbitol à 70% | 1,000 g |
| | - Saccharinate de sodium | 0,010 g |
| | - Parahydroxybenzoate de méthyle | 0,080 g |
| | - Arome | qs |
| | - Eau purifiée | qsp 10 ml |

| B- VOIE PARENTERALE | | |
|---|---|---|
| | a) Composition | |
| | - Composé de l'exemple 3 | 0,002 g |
| | - Oléate d'éthyle | qs 10 g |
| | (b) Composition | |
| | - Composé de l'exemple 1 | 0.05 % |
| | - Polyéthylène glycol | 20% |
| | - Solution de NaCl à 0.9% | qs 100 |
| | (c) Composition | |
| | - Composé de l'exemple 3 | 2.5 % |
| | - Polyéthylène glycol 400 | 20% |
| | - Solution de NaCl à 0.9% | qs 100 |
| | (d) Composition de cyclodextrine injectable | |
| | Composé de l'exemple 3 | 0,1 mg |
| | β- cyclodextrine | 0,10 g |
| | Eau pour injectable | q.s.p.10,00 g |

| C- VOIE TOPIQUE | | |
|---|---|---|
| | (a) Onguent | |
| | - Composé de l'exemple 2 | 0,020 g |
| | - Myristate d'isopropyle | 81,700 g |
| | - Huile de vaseline fluide | 9,100 g |
| | - Silice ("Aérosil 200" vendue par DEGUSSA) | 9,180 g |
| | (b) Onguent | |
| | - Composé de l'exemple 5 | 0,300 g |
| | - Vaseline blanche codex qsp | 100 g |
| | | |
| | (c) Crème Eau-dans-Huile non ionique | |
| | - Composé de l'exemple 4 | 0,100 g |
| | - Mélange d'alcools de lanoline émulsifs, de cires | |
| | et d'huiles ("Eucerine anhydre" vendu par BDF) | 39,900 g |
| | - Parahydroxybenzoate de méthyle | 0,075 g |
| | - Parahydroxybenzoate de propyle | 0,075 g |
| | - Eau déminéralisée stérile qsp | 100 g |
| | (d) Lotion | |
| | - Composé de l'exemple 2 | 0,100 g |
| | - Polyéthylène glycol (PEG 400) | 69,900 g |
| | - Ethanol à 95% | 30,000 g |
| | (e) Onguent hydrophobe | |
| | - Composé de l'exemple 4 | 0,300 g |
| | - Miristate d'isopropyle | 36,400 g |
| | - Huile de silicone ("Rhodorsil 47 V 300" vendu | |
| | par RHONE-POULENC) | 36,400 g |
| | - Cire d'abeille | 13,600 g |
| | - Huile de silicone ("Abil 300.000 cst" vendu | |
| | par GOLDSCHMIDT) | qsp 100 g |
| | (f) Crème Huile-dans-Eau non ionique | |
| | - Composé de l'exemple 6 | 1,000 g |
| | - Alcool cétylique | 4,000 g |
| | - Monostéarate de glycérole | 2,500 g |
| | - Stéarate de PEG 50 | 2,500 g |
| | - Beurre de karité | 9,200 g |
| | - Propylène glycol | 2,000 g |
| | - Parahydroxybenzoate de méthyle | 0,075 g |
| | - Parahydroxybenzoate de propyle | 0,075 g |
| | - Eau déminéralisée stérile | qsp 100 g |

## Revendications

1. Composés **caractérisés par le fait qu'**ils répondent à la formule (I) suivante : 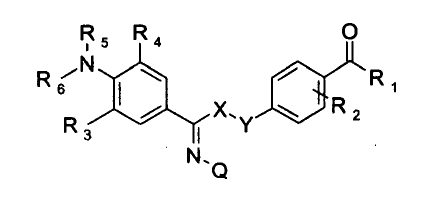 dans laquelle :
- R1 représente un radical -OH, un radical OR7, ou un radical -NR8R9 ;
R7, R8 et R9 ayant les significations données ci-après,
- R2 représente un atome d'hydrogène, un atome de fluor, un atome de chlore, un radical alkyle de 1 à 3 atomes de carbone, un radical -OH, un radical -OR10,
R10 ayant les significations données ci-après,
- R3 représente un atome d'hydrogène, un radical méthyle, un radical éthyle, un radical isopropyle, un radical tertiobutyle, un radical -CF3 ;
- R4 représente un atome d'hydrogène, un atome de fluor, un atome de chlore, un radical -OR11, un radical méthyle, un radical éthyle, un radical isopropyle, un radical tertiobutyle, ou un radical -CF3 ;
R11 ayant les significations données ci-après ;
- R5, R6 identiques ou différents, représentent un atome d'hydrogène, un radical méthyle, un radical éthyle, un radical n-propyle, un radical isopropyle, un radical tertiobutyle, un radical -COR12 ou peuvent former ensemble un groupement piperidine, pyrrolidine, morpholine, pyrrolidin-2-one, ou piperidin-2-one, substitué ou non par un ou plusieurs groupements méthyle, éthyle, fluor, ou chlore ;
R12 ayant les significations données ci-après,
- Q représente un radical -OH, un radical -OR13;
R13, ayant les significations données ci-après; 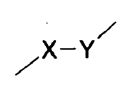 représente une liaison de structure suivante : 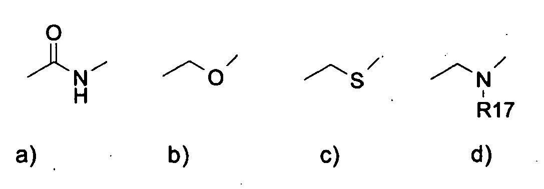 R7 ayant les significations données ci-après,
- R7 représente un radical alkyle, linéaire ou ramifié, ayant de 1 à 20 atomes de carbone, un radical alkényle, un radical monohydroxyalkyle, un radical polyhydroxyalkyle, un radical aryle, un radical aralkyle éventuellement substitué, ou un reste de sucre.
- R8, R9 identiques ou différents, représentent un atome d'hydrogène, un radical -OH, un radical alkyle de 1 à 6 atomes de carbone, un radical monohydroxyalkyle, un radical polyhydroxyalkyle, un radical aryle éventuellement substitué, forment avec l'azote de la structure générale un reste d'aminoacide ou un reste de peptide, ou bien R8 et R9, pris ensemble, forment un groupement piperidine, pyrrolidine, morpholine, pyrrolidin-2-one ou piperidin-2-one;
- R10 représente un radical alkyle ayant de 1 à 6 atomes de carbone;
- R11 représente un radical alkyle ayant de 1 à 6 atomes de carbone, ou un radical mono ou polyéther ;
- R12 représente un radical alkyle ayant de 1 à 6 atomes de carbone ou un groupement -OR18 ;
R18 ayant les significations données ci-après,
- R13 représente un radical alkyle ayant de 1 à 15 atomes de carbone, linéaire ou ramifié ;
- R17 représente un atome d'hydrogène, un radical alkyle ayant de 1 à 6 atomes de carbone, un radical -CF3 ;
- R18 représente un radical alkyle ayant de 1 à 6 atomes de carbone ;
et les sels des composés de formule (I) lorsque R₁ représente une fonction OH ainsi que les isomères optiques et géométriques desdits composés de formule (I).

2. Composés selon la revendication 1, **caractérisés par le fait qu'**ils se présentent sous forme de sels d'un métal alcalin ou alcalino-terreux, de sels de zinc, ou de sels d'une amine organique.

3. Composés selon l'une des revendications 1 ou 2, **caractérisés par le fait que** les radicaux alkyles ayant de 1 à 6 atomes de carbone sont choisis parmi les radicaux méthyle, éthyle, n-propyle, i-propyle, c-propyle isopropyle, n-butyle, i-butyle, t-butyle, n-pentyle, et n-hexyle.

4. Composés selon l'une des revendications 1 à 3, **caractérisés par le fait que** les radicaux alkyles ayant de 1 à 15 atomes de carbone sont choisis parmi les radicaux méthyle, éthyle, propyle, isopropyle, cyclopropyle, n-butyle, i-butyle, t-butyle, n-pentyle, n-hexyle, 2-éthyl-hexyle, octyle et dodécyle.

5. Composés selon l'une des revendications 1 à 4, **caractérisés par le fait que** les radicaux alkyles ayant de 1 à 20 atomes de carbone sont choisis parmi les radicaux méthyle, éthyle, propyle, isopropyle, cyclopropyle, n-butyle, i-butyle, t-butyle, n-pentyle, n-hexyle, 2-éthyl-hexyle, octyle, dodécyle, hexadécyle ou octadécyle.

6. Composés selon l'une des revendications 1 à 5, **caractérisés par le fait que** les radicaux monohydroxyalkyles sont choisis parmi les radicaux hydroxyméthyle, 2-hydroxyéthyle, 2-hydroxypropyle ou 3-hydroxypropyle.

7. Composés selon l'une des revendications 1 à 6, **caractérisés par le fait que** les radicaux polyhydroxyalkyles sont choisis parmi les radicaux 2,3-dihydroxypropyle, 2,3,4-trihydroxybutyle, 2,3,4,5-tétrahydroxypentyle ou le reste du pentaérythritol.

8. Composés selon l'une des revendications 1 à 7, **caractérisés par le fait que** les radicaux polyéthers sont choisis parmi les radicaux méthoxyméthoxy, méthoxyéthoxy ou méthoxyéthoxyméthoxy.

9. Composés selon l'une quelconque des revendications précédentes, **caractérisés par le fait que** les radicaux aryles sont choisis parmi les radicaux phényle éventuellement substitué par au moins un halogène, un alkyle ayant de 1 à 6 atomes de carbone (inférieur), un hydroxyle, un alkoxy en C₁-C₃, une fonction nitro, un radical polyéther ou une fonction amino éventuellement protégée par un groupe acétyle ou éventuellement substitué par au moins un alkyle ayant de 1 à 6 atomes de carbone ou un alkoxy ayant de 1 à 6 atomes de carbone.

10. Composés l'une quelconque des revendications précédentes, **caractérisés par le fait que** les radicaux aralkyles sont choisis parmi les radicaux benzyle ou phénéthyle éventuellement substitué par au moins un halogène, un alkyle ayant de 1 à 6 atomes de carbone, un hydroxyle, un radical alkoxy ayant de 1 à 3 atomes de carbone, une fonction nitro, un radical polyéther ou une fonction amino éventuellement protégée par un groupe acétyle ou éventuellement substitué par au moins un alkyle ayant de 1 à 6 atomes de carbone ou un radical alkoxy ayant de 1 à 6 atomes de carbone.

11. Composés selon l'une quelconque des revendications précédentes, **caractérisés par le fait que** les radicaux alkényles sont choisis parmi les radicaux ayant de préférence 2 à 5 atomes de carbone et présentant une ou plusieurs insaturations éthyléniques.

12. Composés selon l'une quelconque des revendications précédentes, **caractérisés par le fait que** le reste de sucre est choisi dans le groupe constitué par le reste dérivant du glucose, du galactose ou du mannose, ou bien encore de l'acide glucuronique.

13. Composés selon l'une quelconque des revendications précédentes, **caractérisés par le fait que** le reste d'aminoacide est choisi dans le groupe constitué par un reste dérivant de la lysine, de la glycine ou de l'acide aspartique.

14. Composés selon l'une quelconque des revendications précédentes, **caractérisés par le fait que** le reste d'aminoacide est choisi dans le groupe constitué par un reste de dipeptides ou de tripeptides résultant de la combinaison d'acides aminés.

15. Composés de formule (1) selon l'une quelconque des revendications précédentes choisis parmi:
1. Acide 4-[2-(3-*tert*-butyl-4-diethylamino-phenyl)-2-hydroxyimino-ethoxy]-benzoïque ;
2. Acide 4-[2-(3-*tert*-butyl-4-diethylamino-phenyl)-2-hydroxyimino-ethoxy]-2-hydroxy-benzoïque ;
3. Acide 4-[2-(3-*tert*-butyl-4-pyrrolidin-1-yl-phenyl)-2-hydroxyimino-ethoxy]-benzoïque ;
4. Acide 4-[2-(3-*tert*-butyl-4-pyrrolidin-1-yl-phenyl)-2-hydroxyimino-ethoxy]-2-hydroxy-benzoïque ;
5. Acide 4-{2-[4-(acetyl-ethyl-amino)-3-*tert*-butyl-phenyl]-2-hydroxyimino-ethoxy}-benzoïque ;
6. Acide 4-{2-[4-(acetyl-ethyl-amino)-3-*tert*-butyl-phenyl]-2-hydroxyimino-ethoxy}-2-hydroxy-benzoïque ;
7. 4-[2-(3-*tert*-Butyl-4-diethylamino-phenyl)-2-hydroxyimino-ethoxy]-2-hydroxy-benzoate d'éthyle ;
8. 4-[2-(3-*tert*-Butyl-4-diethylamino-phenyl)-2-hydroxyimino-ethoxy]-2-hydroxy-benzoate d'isopropyle ;
9. 4-[2-(3-*tert*-Butyl-4-diethylamino-phenyl)-2-hydroxyimino-ethoxy]-2-hydroxy-benzoate d'isobutyle ;
10. 4-[2-(3-*tert*-Butyl-4-diethylamino-phenyl)-2-hydroxyimino-ethoxy]-2-hydroxy-benzoate d'octyle ;
11. 4-[2-(3-*tert*-Butyl-4-diethylamino-phenyl)-2-hydroxyimino-ethoxy]-2-hydroxy-benzoate de dodecyle ;
12. 1-(3-*tert*-Butyl-4-diethylamino-phenyl)-2-[3-hydroxy-4-(morpholine-4-carbonyl)-phenoxy]-ethanone oxime ;
13. 4-[2-(3-*tert*-Butyl-4-diethylamino-phenyl)-2-hydroxyimino-ethoxy]-N-ethyl-2-hydroxy-N-methyl-benzamide ;
14. Acide 4-[2-(3-*tert*-butyl-4-diethylamino-phenyl)-2-hydroxyimino-ethylsulfanyl]-2-hydroxy-benzoïque ;
15. Acide 4-[2-(3-*tert*-butyl-4-diethylamino-phenyl)-2-hydroxyimino-ethylsulfanyl]-benzoïque ;
16. Acide 4-[2-(3-*tert*-Butyl-4-diethylamino-phenyl)-2-hydroxyimino-ethylamino]-2-hydroxy-benzoïque ;
17. Acide 4-{[2-(3-*tert*-Butyl-4-diethylamino-phenyl)-2-hydroxyimino-ethyl]-methyl-amino}-2-hydroxy- benzoïque ;
18. Acide 4-[3-(3-*tert*-Butyl-4-diethylamino-phenyl)-3-hydroxyimino-propyl]-2-hydroxy-benzoïque ;
19. Acide 4-[2-(3-*tert*-Butyl-4-diethylamino-phenyl)-2-hydroxyimino-acetylamino]-2-hydroxy-benzoïque ;
20. Acide 4-[2-(3-*tert*-Butyl-4-diethylamino-phenyl)-2-hydroxyimino-acetylamino]-benzoïque ;
21. Acide 4-[2-(3-*tert*-Butyl-4-diethylamino-phenyl)-2-methoxyimino-acetylamino]-2-hydroxy- benzoïque ;
22. Acide 4-[2-(3-*tert*-Butyl-4-diethylamino-phenyl)-2-methoxyimino-ethoxy]-2-hydroxy-benzoïque ;
23. Acide 4-[2-(3-tert-Butyl-5-chloro-4-diethylamino-phenyl)-2-hydroxyimino-ethoxy]-2-hydroxy- benzoïque ;
24. Acide 4-[2-(3-tert-Butyl -4-diethylamino-5-fluoro -phenyl)-2-hydroxyimino-ethoxyl-2-hydroxy- benzoïque ;
25. Acide 4-[2-(3-tert-Butyl-4-diethylamino-5-trifluoromethyl-phenyl)-2-hydroxyimino-ethoxy]-2-hydroxy- benzoïque ;
26. Acide 4-[2-(3-tert-Butyl -4-diethylamino-5-methyl-phenyl)-2-hydroxyimino-ethoxy]-2-hydroxy- benzoïque ;
27. Acide 4-[2-(3-tert-Butyl -4-diethylamino-5-ethyl-phenyl)-2-hydroxyimino-ethoxy]-2-hydroxy- benzoïque ;
28. Acide 4-{2-[3-tert-Butyl-4-diethylamino-5-(2-ethoxy-ethoxy)-phenyl]-2-hydroxyimino-ethoxy}-2-hydroxy- benzoïque ;
29. Acide 4-[2-(3-tert-Butyl -4-diethylamino-5-propoxy-phenyl)-2-hydroxyimino-ethoxy]-2-hydroxy- benzoïque ;
30. Acide 4-[2-(4-diethylamino-3-iso-propyl-phenyl)-2-hydroxyimino-ethoxy]-2-hydroxy-benzoïque ;
31. Acide 4-[2-(4-diethylamino-3-trifluoromethyl-phenyl)-2-hydroxyimino-ethoxy]-2-hydroxy-benzoïque ;
32. Acide 4-[2-(4-diethylamino-phenyl)-2-hydroxyimino-ethoxy]-2-hydroxy-benzoïque ;
33. Acide 4-{2-[3-tert-butyl-4-(ethyl-methyl-amino)-phenyl]-2-hydroxyimino-ethoxy}-2-hydroxy-benzoïque ;
34. Acide 4-[2-(3-*tert*-butyl-4-dimethylamino-phenyl)-2-hydroxyimino-ethoxy]-2-hydroxy-benzoïque ;
35. Acide 4-{2-[3-tert-butyl-4-(ethyl-*iso-*butyl-amino)-phenyl]-2-hydroxyimino-ethoxy}-2-hydroxy-benzoïque ;
36. Acide 4-[2-(3-tert-butyl-4-morpholin-4-yl-phenyl)-2-hydroxyimino-ethoxy]-2-hydroxy-benzoïque ;
37. Acide 4-[2-(3-tert-butyl-4-piperidin-1-yl-phenyl)-2-hydroxyimino-ethoxy]-2-hydroxy-benzoïque ;
38. Acide 4-{2-[3-tert-butyl-4-(2-oxo-piperidin-1-yl)-phenyl]-2-hydroxyimino-ethoxy}-benzoïque ;
39. Acide 4-{2-[3-tert-butyl-4-(2-oxo-pyrrolidin-1-yl)-phenyl]-2-hydroxyimino-ethoxy}-benzoïque ;
40. Acide 4-[2-(3-*tert*-butyl-4-ethylamino-phenyl)-2-hydroxyimino-ethoxy]-2-hydroxy-benzoïque ;
41. Acide 4-{2-[4-(ethyl-propionyl-amino)-3-*tert*-butyl-phenyl]-2-hydroxyimino-ethoxy}-2-hydroxy-benzoïque ;
42. Acide 4-{2-[3-tert-butyl-4-(ethoxycarbonyl-ethyl-amino)-phenyl]-2-hydroxyimino-ethoxy}-2-hydroxy-benzoïque ;
43. Acide 4-{2-[3-tert-butyl-4-(ethyl-methoxycarbonyl-amino)-phenyl]-2-hydroxyimino-ethoxy}-2-hydroxy-benzoïque ;
44. Acide 4-{2-[4-(ethyl-trifluoroacetyl-amino)-3-tert-butyl-phenyl]-2-hydroxyimino-ethoxy}-2-hydroxy-benzoïque ;
45. Acide 4-{2-[4-(trifluoroacetyl-amino)-3-*tert*-butyl-phenyl]-2-hydroxyimino-ethoxy}-2-hydroxy-benzoïque ;
46. Acide 4-{2-[3-tert-butyl-4-(ethyl-isopropyl-amino)-phenyl]-2-hydroxyimino-ethoxy}-2-hydroxy-benzoïque.

16. Composés selon la revendication 1 ou 2, **caractérisés par le fait qu'**ils présentent une des caractéristiques suivantes :
- R1 représente le radical -OR7 ;
- R2 représente un hydrogène ou un -OH ;
- R5, R6 identiques ou différents représentent un radical méthyle ou un radical éthyle ou forment ensemble un groupement pyrrolidine ;
- Q représente un radical -OR13.

17. Composés selon l'une quelconque des revendications 1 à 16 à titre de médicament.

18. Utilisation d'un composé selon l'une quelconque des revendications 1 à 16 dans la fabrication d'une composition destinée au traitement :
- des affections dermatologiques liées à un désordre de la kératinisation portant sur la différenciation et sur la prolifération cellulaire ;
- des ichtyoses, des états ichtyosiformes, de la maladie de Darrier, des kératodermies palmoplantaires, des leucoplasies et des états leucoplasiformes, du lichen cutané ou muqueux (buccal) ;
- des affections dermatologiques avec une composante immuno-allergique inflammatoire, avec ou sans trouble de la prolifération cellulaire ;
- des proliférations dermiques ou épidermiques bénignes ou malignes, d'origine virale ou non ;
- des proliférations pouvant être induites par les ultra-violets ;
- des lésions précancéreuses cutanées ;
- des dermatoses immunes ;
- des maladies immunes bulleuses ;
- des maladies du collagène;
- des affections dermatologiques à composante immunologique ;
- des troubles ophtalmologiques;
- des stigmates de l'atrophie épidermique et/ou dermique induite par les corticostéroïdes locaux ou systémiques, ou toute autre forme d'atrophie cutanée;
- des affections d'origine virale au niveau cutané;
- de désordres cutanés dus à une exposition aux rayonnements U.V, du vieillissement de la peau, photo-induit ou chronologique ou des pigmentations et des kératoses actiniques ;
- des pathologies associées au vieillissement chronologique ou actinique de la peau ;
- des troubles de la fonction sébacée ;
- des troubles de la cicatrisation ou des vergetures ; ou
- des désordres de la pigmentation.

19. Composition pharmaceutique, **caractérisée par le fait qu'**elle comprend, dans un support physiologiquement acceptable, au moins l'un des composés tels que définis à l'une quelconque des revendications 1 à 16.

20. Composition selon la revendication 19, **caractérisée en ce que** la concentration en composé(s) selon l'une des revendications 1 à 16 est comprise entre 0,001% et 10% en poids par rapport au poids total de la composition.

21. Composition selon la revendication 20, **caractérisée en ce que** la concentration en composé(s) selon l'une des revendications 1 à 16 est comprise entre 0, 01% et 1% en poids par rapport au poids total de la composition.

22. Composition cosmétique, **caractérisée par le fait qu'**elle comprend, dans un milieu physiologiquement acceptable, au moins un des composés tels que définis à l'une quelconque des revendications 1 à 16.

23. Composition selon la revendication 22, **caractérisée en ce que** la concentration en composé(s) selon l'une des revendications 1 à 16 est comprise entre 0,001% et 3% en poids par rapport au poids total de la composition.

24. Utilisation non thérapeutique d'une composition cosmétique telle que définie dans l'une des revendications 22 ou 23 pour prévenir et/ou traiter les signes du vieillissement et/ou la peau sèche.

25. Utilisation non thérapeutique d'une composition cosmétique telle que définie à l'une des revendications 22 ou 23 pour l'hygiène corporelle ou capillaire.

26. Procédé cosmétique d'embellissement de la peau, **caractérisé par le fait que** l'on applique sur la peau une composition telle que définie dans l'une quelconque des revendications 22 à 23.

## Claims

1. Compounds **characterized in that** they correspond to formula (I) below: 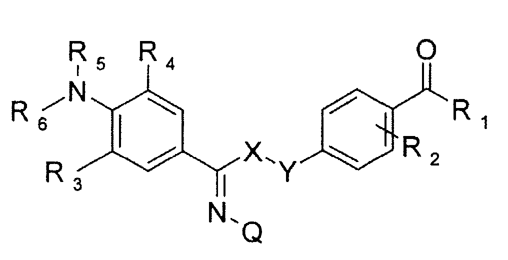 in which:
- R1 represents an -OH radical, a radical OR7 or a radical -NR8R9 ;
R7, R8 and R9 having the meanings given below,
- R2 represents a hydrogen atom, a fluorine atom, a chlorine atom, an alkyl radical of 1 to 3 carbon atoms,
an -OH radical or a radical -OR10,
R10 having the meanings given below,
- R3 represents a hydrogen atom, a methyl radical, an ethyl radical, an isopropyl radical, a tert-butyl radical or a -CF3 radical;
- R4 represents a hydrogen atom, a fluorine atom, a chlorine atom, a radical -OR11, a methyl radical, an ethyl radical, an isopropyl radical, a tert-butyl radical or a -CF3 radical;
R11 having the meanings given below;
- R5 and R6, which may be identical or different, represent a hydrogen atom, a methyl radical, an ethyl radical, an n-propyl radical, an isopropyl radical, a tert-butyl radical, a radical -COR12 or may together form a piperidine, pyrrolidine, morpholine, pyrrolidin-2-one or piperidin-2-one group, which is unsubstituted or substituted with one or more methyl, ethyl, fluorine or chlorine groups;
R12 having the meanings given below,
- Q represents an -OH radical or a radical -OR13 ;
R13 having the meanings given below; 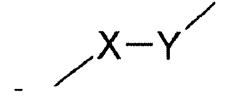 represents a bond having the following structure: 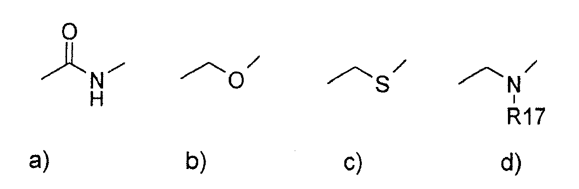 R7 having the meanings given below,
- R7 represents a linear or branched alkyl radical containing from 1 to 20 carbon atoms, an alkenyl radical, a monohydroxyalkyl radical, a polyhydroxyalkyl radical, an aryl radical, an optionally substituted aralkyl radical or a sugar residue,
- R8 and R9, which may be identical or different, represent a hydrogen atom, an -OH radical, an alkyl radical of 1 to 6 carbon atoms, a monohydroxyalkyl radical, a polyhydroxyalkyl radical, an optionally substituted aryl radical, or form with the nitrogen of the general structure an amino acid residue or a peptide residue, or alternatively R8 and R9, taken together, form a piperidine, pyrrolidine, morpholine, pyrrolidin-2-one or piperidin-2-one group;
- R10 represents an alkyl radical containing from 1 to 6 carbon atoms;
- R11 represents an alkyl radical containing from 1 to 6 carbon atoms or a mono- or polyether radical;
- R12 represents an alkyl radical containing from 1 to 6 carbon atoms or a group -OR18 ;
R18 having the meanings given below,
- R13 represents a linear or branched alkyl radical containing from 1 to 15 carbon atoms;
- R17 represents a hydrogen atom, an alkyl radical containing from 1 to 6 carbon atoms or a -CF3 radical;
- R18 represents an alkyl radical containing from 1 to 6 carbon atoms;
and the salts of the compounds of formula (I) when R₁ represents an OH function, and also the optical and geometrical isomers of the said compounds of formula (I).

2. Compounds according to Claim 1, **characterized in that** they are in the form of salts of an alkali metal or alkaline-earth metal, zinc salts or salts of an organic amine.

3. Compounds according to either of Claims 1 and 2, **characterized in that** the alkyl radicals containing from 1 to 6 carbon atoms are chosen from methyl, ethyl, n-propyl, i-propyl, c-propyl, isopropyl, n-butyl, i-butyl, t-butyl, n-pentyl and n-hexyl radicals.

4. Compounds according to one of Claims 1 to 3, **characterized in that** the alkyl radicals containing from 1 to 15 carbon atoms are chosen from methyl, ethyl, propyl, isopropyl, cyclopropyl, n-butyl, i-butyl, t-butyl, n-pentyl, n-hexyl, 2-ethylhexyl, octyl and dodecyl radicals.

5. Compounds according to one of Claims 1 to 4, **characterized in that** the alkyl radicals containing from 1 to 20 carbon atoms are chosen from methyl, ethyl, propyl, isopropyl, cyclopropyl, n-butyl, i-butyl, t-butyl, n-pentyl, n-hexyl, 2-ethylhexyl, octyl, dodecyl, hexadecyl and octadecyl radicals.

6. Compounds according to one of Claims 1 to 5, **characterized in that** the monohydroxyalkyl radicals are chosen from hydroxymethyl, 2-hydroxyethyl, 2-hydroxypropyl and 3-hydroxypropyl radicals.

7. Compounds according to one of Claims 1 to 6, **characterized in that** the polyhydroxyalkyl radicals are chosen from 2,3-dihydroxypropyl, 2,3,4-trihydroxybutyl and 2,3,4,5-tetrahydroxypentyl radicals or a pentaerythritol residue.

8. Compounds according to one of Claims 1 to 7, **characterized in that** the polyether radicals are chosen from methoxymethoxy, methoxyethoxy and methoxyethoxymethoxy radicals.

9. Compounds according to any one of the preceding claims, **characterized in that** the aryl radicals are chosen from a phenyl radical optionally substituted with at least one halogen, an (a lower) alkyl containing from 1 to 6 carbon atoms, a hydroxyl, a C₁-C₃ alkoxy, a nitro function, a polyether radical or an amino function optionally protected with an acetyl group or optionally substituted with at least one alkyl containing from 1 to 6 carbon atoms or an alkoxy containing from 1 to 6 carbon atoms.

10. Compounds according to any one of the preceding claims, **characterized in that** the aralkyl radicals are chosen from a benzyl or phenethyl radical optionally substituted with at least one halogen, an alkyl containing from 1 to 6 carbon atoms, a hydroxyl, an alkoxy radical containing from 1 to 3 carbon atoms, a nitro function, a polyether radical or an amino function optionally protected with an acetyl group or optionally substituted with at least one alkyl containing from 1 to 6 carbon atoms or an alkoxy radical containing from 1 to 6 carbon atoms.

11. Compounds according to any one of the preceding claims, **characterized in that** the alkenyl radicals are chosen from radicals preferably containing from 2 to 5 carbon atoms and having one or more ethylenic unsaturations.

12. Compounds according to any one of the preceding claims, **characterized in that** the sugar residue is chosen from the group consisting of the residue derived from glucose, galactose or mannose, or alternatively from glucuronic acid.

13. Compounds according to any one of the preceding claims, **characterized in that** the amino acid residue is chosen from the group consisting of a residue derived from lysine, from glycine or from aspartic acid.

14. Compounds according to any one of the preceding claims, **characterized in that** the amino acid residue is chosen from the group consisting of a dipeptide or tripeptide residue resulting from the combination of amino acids.

15. Compounds of formula (1) according to any one of the preceding claims, chosen from:
1. 4-[2-(3-*tert*-Butyl-4-diethylaminophenyl)-2-hydroxyiminoethoxy]benzoic acid;
2. 4-[2-(3-*tert*-Butyl-4-diethylaminophenyl)-2-hydroxyiminoethoxy]-2-hydroxybenzoic acid;
3. 4-[2-(3-*tert*-Butyl-4-pyrrolidin-1-ylphenyl)-2-hydroxyiminoethoxy]benzoic acid;
4. 4-[2-(3-*tert*-Butyl-4-pyrrolidin-1-ylphenyl)-2-hydroxyiminoethoxy]-2-hydroxybenzoic acid;
5. 4-{2-[4-(Acetylethylamino)-3-*tert*-butylphenyl]-2-hydroxyiminoethoxy}benzoic acid;
6. 4-{2-[4-(Acetylethylamino)-3-*tert*-butylphenyl]-2-hydroxyiminoethoxy}-2-hydroxybenzoic acid;
7. Ethyl 4-[2-(3-*tert*-butyl-4-diethylaminophenyl)-2-hydroxyiminoethoxy]-2-hydroxybenzoate;
8. Isopropyl 4-[2-(3-*tert*-butyl-4-diethylaminophenyl)-2-hydroxyiminoethoxy]-2-hydroxybenzoate;
9. Isobutyl 4-[2-(3-*tert*-butyl-4-diethylaminophenyl)-2-hydroxyiminoethoxy]-2-hydroxybenzoate;
10. Octyl 4-[2-(3-*tert*-butyl-4-diethylaminophenyl)-2-hydroxyiminoethoxy]-2-hydroxybenzoate;
11. Dodecyl 4-[2-(3-*tert*-butyl-4-diethylaminophenyl)-2-hydroxyiminoethoxy]-2-hydroxybenzoate;
12. 1-(3-*tert*-Butyl-4-diethylaminophenyl)-2-[3-hydroxy-4-(morpholine-4-carbonyl)phenoxy]ethanone oxime;
13. 4-[2-(3-*tert*-Butyl-4-diethylaminophenyl)-2-hydroxyiminoethoxy]-N-ethyl-2-hydroxy-N-methyl-benzamide;
14. 4-[2-(3-*tert*-Butyl-4-diethylaminophenyl)-2-hydroxyiminoethylsulfanyl]-2-hydroxybenzoic acid;
15. 4-[2-(3-*tert*-Butyl-4-diethylaminophenyl)-2-hydroxyiminoethylsulfanyl]benzoic acid;
16. 4-[2-(3-*tert*-Butyl-4-diethylaminophenyl)-2-hydroxyiminoethylamino]-2-hydroxybenzoic acid;
17. 4-{[2-(3-*tert*-Butyl-4-diethylaminophenyl)-2-hydroxyiminoethyl]methylamino}-2-hydroxybenzoic acid;
18. 4-[3-(3-*tert*-Butyl-4-diethylaminophenyl)-3-hydroxyiminopropyl]-2-hydroxybenzoic acid;
19. 4-[2-(3-*tert*-Butyl-4-diethylaminophenyl)-2-hydroxyiminoacetylamino]-2-hydroxybenzoic acid;
20. 4-[2-(3-*tert*-Butyl-4-diethylaminophenyl)-2-hydroxyiminoacetylamino]benzoic acid;
21. 4-[2-(3-*tert*-Butyl-4-diethylaminophenyl)-2-methoxyiminoacetylamino]-2-hydroxybenzoic acid;
22. 4-[2-(3-*tert*-Butyl-4-diethylaminophenyl)-2-methoxyiminoethoxy]-2-hydroxybenzoic acid;
23. 4-[2-(3-*tert*-Butyl-5-chloro-4-diethylaminophenyl)-2-hydroxyiminoethoxy]-2-hydroxybenzoic acid;
24. 4-[2-(3-*tert*-Butyl-4-diethylamino-5-fluorophenyl)-2-hydroxyiminoethoxy]-2-hydroxybenzoic acid;
25. 4-[2-(3-*tert*-Butyl-4-diethylamino-5-trifluoromethylphenyl)-2-hydroxyiminoethoxy]-2-hydroxybenzoic acid;
26. 4-[2-(3-*tert*-Butyl-4-diethylamino-5-methylphenyl)-2-hydroxyiminoethoxy]-2-hydroxybenzoic acid;
27. 4-[2-(3-*tert*-Butyl-4-diethylamino-5-ethylphenyl)-2-hydroxyiminoethoxy]-2-hydroxybenzoic acid;
28. 4-{2-[3-*tert*-Butyl-4-diethylamino-5-(2-ethoxy-ethoxy)phenyl]-2-hydroxyiminoethoxy}-2-hydroxybenzoic acid;
29. 4-[2-(3-*tert*-Butyl-4-diethylamino-5-propoxyphenyl)-2-hydroxyiminoethoxy]-2-hydroxybenzoic acid;
30. 4-[2-(4-diethylamino-3-isopropylphenyl)-2-hydroxyiminoethoxy]-2-hydroxybenzoic acid;
31. 4-[2-(4-diethylamino-3-trifluoromethylphenyl)-2-hydroxyiminoethoxy]-2-hydroxybenzoic acid;
32. 4-[2-(4-diethylaminophenyl)-2-hydroxyiminoethoxy]-2-hydroxybenzoic acid;
33. 4-{2-[3-*tert*-Butyl-4-(ethylmethylamino)phenyl]-2-hydroxyiminoethoxy}-2-hydroxybenzoic acid;
34. 4-[2-(3-*tert*-Butyl-4-dimethylaminophenyl)-2-hydroxyiminoethoxy]-2-hydroxybenzoic acid;
35. 4-{2-[3-*tert*-Butyl-4-(ethylisobutylamino)phenyl]-2-hydroxyiminoethoxy}-2-hydroxybenzoic acid;
36. 4-[2-(3-*tert*-Butyl-4-morpholin-4-ylphenyl)-2-hydroxyiminoethoxy]-2-hydroxybenzoic acid;
37. 4-[2-(3-*tert*-Butyl-4-piperidin-1-ylphenyl)-2-hydroxyiminoethoxy]-2-hydroxybenzoic acid;
38. 4-{2-[3-*tert*-Butyl-4-(2-oxopiperidin-1-yl)phenyl]-2-hydroxyiminoethoxy}benzoic acid;
39. 4-{2-[3-*tert*-Butyl-4-(2-oxopyrrolidin-1-yl)-phenyl]-2-hydroxyiminoethoxy}benzoic acid;
40. 4-[2-(3-*tert*-Butyl-4-ethylaminophenyl)-2-hydroxy-iminoethoxy]-2-hydroxybenzoic acid;
41. 4-{2-[4-(Ethylpropionylamino)-3-*tert*-butylphenyl]-2-hydroxyiminoethoxy}-2-hydroxybenzoic acid;
42. 4-{2-[3-*tert*-Butyl-4-(ethoxycarbonylethylamino)-phenyl]-2-hydroxyiminoethoxy}-2-hydroxybenzoic acid;
43. 4-{2-[3-*tert*-Butyl-4-(ethylmethoxycarbonylamino)-phenyl]-2-hydroxyiminoethoxy}-2-hydroxybenzoic acid;
44. 4-{2-[4-(Ethyltrifluoroacetylamino)-3-tert-butylphenyl]-2-hydroxyiminoethoxy}-2-hydroxybenzoic acid;
45. 4-{2-[4-(Trifluoroacetylamino)-3-tert-butylphenyl]-2-hydroxyiminoethoxy}-2-hydroxybenzoic acid;
46. 4-{2-[3-*tert*-Butyl-4-(ethylisopropylamino)phenyl]-2-hydroxyiminoethoxy}-2-hydroxybenzoic acid.

16. Compounds according to Claim 1 or 2, **characterized in that** they have one of the following characteristics:
- R1 represents the radical -OR7;
- R2 represents a hydrogen or an -OH;
- R5 and R6, which may be identical or different, represent a methyl radical or an ethyl radical, or together form a pyrrolidine group;
- Q represents a radical -OR13.

17. Compounds according to any one of Claims 1 to 16, as medicinal products.

18. Use of a compound according to any one of Claims 1 to 16 in the manufacture of a composition for treating:
- dermatological complaints associated with a keratinization disorder relating to cell differentiation and proliferation;
- ichthyosis, ichthyosiform conditions, Darier's disease, palmoplantar keratoderma, leukoplakia and leukoplakiform conditions, and cutaneous or mucous (buccal) lichen;
- dermatological complaints with an inflammatory immunoallergic component, with or without a cell proliferation disorder;
- benign or malignant dermal or epidermal proliferations, of viral or non-viral origin;
- proliferations that may be induced by ultraviolet radiation;
- precancerous skin lesions;
- immune dermatoses;
- immune bullous diseases;
- collagen diseases;
- dermatological complaints with an immunological component;
- ophthalmological disorders;
- stigmata of epidermal and/or dermal atrophy induced by local or systemic corticosteroids, or any other form of cutaneous atrophy;
- skin complaints of viral origin;
- skin disorders caused by exposure to UV radiation, photoinduced or chronological ageing of the skin, or actinic pigmentations and keratoses;
- pathologies associated with chronological or actinic ageing of the skin;
- disorders of sebaceous function;
- cicatrization disorders or stretch marks; or
- pigmentation disorders.

19. Pharmaceutical composition, **characterized in that** it comprises, in a physiologically acceptable support, at least one of the compounds as defined in any one of Claims 1 to 16.

20. Composition according to Claim 19, **characterized in that** the concentration of compound(s) according to one of Claims 1 to 16 is between 0.001% and 10% by weight, relative to the total weight of the composition.

21. Composition according to Claim 20, **characterized in that** the concentration of compound(s) according to one of Claims 1 to 16 is between 0.01% and 1% by weight, relative to the total weight of the composition.

22. Cosmetic composition, **characterized in that** it comprises, in a physiologically acceptable medium, at least one of the compounds as defined in any one of Claims 1 to 16.

23. Composition according to Claim 22, **characterized in that** the concentration of compound(s) according to one of Claims 1 to 16 is between 0.001% and 3% by weight relative to the total weight of the composition.

24. Non-therapeutic use of a cosmetic composition as defined in either of Claims 22 and 23 for preventing and/or treating the signs of ageing and/or dry skin.

25. Non-therapeutic use of a cosmetic composition as defined in either of Claims 22 and 23, for body or hair hygiene.

26. Cosmetic process for enhancing the appearance of the skin, **characterized in that** a composition as defined in either of Claims 22 and 23 is applied to the skin.

## Patentansprüche

1. Verbindungen, **dadurch gekennzeichnet, dass** sie der folgenden Formel (I) entsprechen: 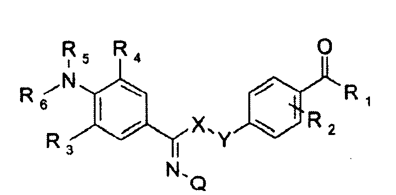 in der Formel:
- R1 bedeutet eine Gruppe -OH, eine Gruppe OR7 oder eine Gruppe -NR8R9, wobei R7, R8 und R9 die nachstehend angegebenen Bedeutungen aufweisen;
- R2 bedeutet ein Wasserstoffatom, ein Fluoratom, ein Chloratom, eine Alkylgruppe mit 1 bis 3 Kohlenstoffatomen, eine Gruppe -OH, eine Gruppe -OR10, wobei R10 die nachstehend angegebenen Bedeutungen aufweist;
- R3 bedeutet ein Wasserstoffatom, eine Methylgruppe, eine Ethylgruppe, eine Isopropylgruppe, eine tert-Butylgruppe, eine Gruppe -CF3;
- R4 bedeutet ein Wasserstoffatom, ein Fluoratom, ein Chloratom, eine Gruppe -OR11, eine Methylgruppe, eine Ethylgruppe, eine Isopropylgruppe, eine tert-Butylgruppe oder eine Gruppe -CF3, wobei R11 die nachstehend angegebenen Bedeutungen aufweist;
- R5 und R6, die gleich oder verschieden sind, bedeuten ein Wasserstoffatom, eine Methylgruppe, eine Ethylgruppe, eine n-Propylgruppe, eine Isopropylgruppe, eine tert-Butylgruppe oder eine Gruppe -COR12 oder sie können gemeinsam eine Piperidingruppe, Pyrrolidingruppe, Morpholingruppe, Pyrrolidin-2-ongruppe oder Piperidin-2-ongruppe bilden, die gegebenenfalls mit einer oder mehreren Gruppen Methyl, Ethyl, Fluor oder Chlor substituiert sind, wobei R12 die nachstehend angegebenen Bedeutungen aufweist;
- Q bedeutet eine Gruppe -OH, eine Gruppe -OR13, wobei R13 die nachstehend angegebenen Bedeutungen aufweist; 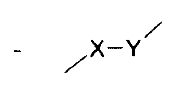 bedeutet eine Bindung der folgenden Struktur: 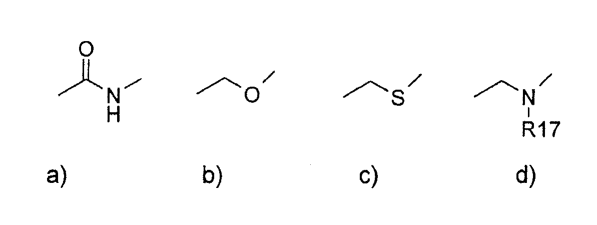 wobei R7 die nachstehend angegebenen Bedeutungen aufweist;
- R7 bedeutet eine lineare oder verzweigte Alkylgruppe mit 1 bis 20 Kohlenstoffatomen, eine Alkenylgruppe, eine Monohydroxyalkylgruppe, eine Polyhydroxyalkylgruppe, eine Arylgruppe, eine Aralkylgruppe, die gegebenenfalls substituiert ist, oder einen Zuckerrest;
- R8 und R9, die gleich oder verschieden sind, bedeuten ein Wasserstoffatom, eine Gruppe -OH, eine Alkylgruppe mit 1 bis 6 Kohlenstoffatomen, eine Monohydroxyalkylgruppe, eine Polyhydroxyalkylgruppe, eine Arylgruppe, die gegebenenfalls substituiert ist, sie bilden mit dem Stickstoffatom der allgemeinen Struktur einen Aminosäurerest oder einen Peptidrest oder R8 und R9 bilden gemeinsam eine Gruppe Piperidin, Pyrrolidin, Morpholin, Pyrrolidin-2-on oder Piperidin-2-on;
- R10 bedeutet eine Alkylgruppe mit 1 bis 6 Kohlenstoffatomen;
- R11 bedeutet eine Alkylgruppe mit 1 bis 6 Kohlenstoffatomen oder einen Mono- oder Polyether;
- R 12 bedeutet eine Alkylgruppe mit 1 bis 6 Kohlenstoffatomen oder eine Gruppe -OR18, wobei R18 die nachstehend angegebenen Bedeutungen aufweist;
- R13 bedeutet eine lineare oder verzweigte Alkylgruppe mit 1 bis 15 Kohlenstoffatomen;
- R17 bedeutet ein Wasserstoffatom, eine Alkylgruppe mit 1 bis 6 Kohlenstoffatomen oder eine Gruppe -CF3 ;
- R18 bedeutet eine Alkylgruppe mit 1 bis 6 Kohlenstoffatomen;
und die Salze der Verbindungen der Formel (I), wenn R1 eine OH-Funktion bedeutet, sowie die optischen und geometrischen Isomere der Verbindungen der Formel (I).

2. Verbindungen nach Anspruch 1, **dadurch gekennzeichnet, dass** sie in der Form von Salzen eines Alkali- oder Erdalkalimetalls, Zinksalzen oder Salzen eines organischen Amins vorliegen.

3. Verbindungen nach einem der Ansprüche 1 oder 2, **dadurch gekennzeichnet, dass** die Alkylgruppen mit 1 bis 6 Kohlenstoffatomen unter den Gruppen Methyl, Ethyl, n-Propyl, i-Propyl, Cyclopropyl, Isopropyl, n-Butyl, Isobutyl, tert-Butyl, n-Pentyl und n-Hexyl ausgewählt sind.

4. Verbindungen nach einem der Ansprüche 1 bis 3, **dadurch gekennzeichnet, dass** die Alkylgruppen mit 1 bis 15 Kohlenstoffatomen unter den Gruppen Methyl, Ethyl, Propyl, Isopropyl, Cyclopropyl, n-Butyl, Isobutyl, tert-Butyl, n-Pentyl, n-Hexyl, 2-Ethyl-hexyl, Octyl und Dodecyl ausgewählt sind.

5. Verbindungen nach einem der Ansprüche 1 bis 4, **dadurch gekennzeichnet, dass** die Alkylgruppen mit 1 bis 20 Kohlenstoffatomen unter den Gruppen Methyl, Ethyl, Propyl, Isopropyl, Cyclopropyl, n-Butyl, Isobutyl, tert-Butyl, n-Pentyl, n-Hexyl, 2-Ethyl-hexyl, Octyl, Dodecyl, Hexadecyl oder Octadecyl ausgewählt sind.

6. Verbindungen nach einem der Ansprüche 1 bis 5, **dadurch gekennzeichnet, dass** die Monohydroxyalkylgruppen unter den Gruppen Hydroxymethyl, 2-Hydroxyethyl, 2-Hydroxypropyl oder 3-Hydroxypropyl ausgewählt sind.

7. Verbindungen nach einem der Ansprüche 1 bis 6, **dadurch gekennzeichnet, dass** die Polyhydroxyalkylgruppen unter den Gruppen 2,3-Dihydroxypropyl, 2,3,4-Trihydroxybutyl, 2,3,4,5-Tetrahydroxypentyl oder dem Pentaerythritrest ausgewählt sind.

8. Verbindungen nach einem der Ansprüche 1 bis 7, **dadurch gekennzeichnet, dass** die Polyethergruppen unter den Gruppen Methoxymethoxy, Methoxyethoxy oder Methoxyethoxymethoxy ausgewählt sind.

9. Verbindungen nach einem der vorhergehenden Ansprüche, **dadurch gekennzeichnet, dass** die Arylgruppen unter den Phenylgruppen ausgewählt sind, die gegebenenfalls substituiert sind mit mindestens einem Halogenatom, einer (niederen) Alkylgruppe mit 1 bis 6 Kohlenstoffatomen, einer Hydroxygruppe, einer Alkoxygruppe mit 1 bis 3 Kohlenstoffatomen, einer Nitrofunktion, einer Polyethergruppe oder einer Aminogruppe, die gegebenenfalls mit Acetyl geschützt ist oder gegebenenfalls mit mindestens einer Alkylgruppe mit 1 bis 6 Kohlenstoffatomen oder einer Alkoxygruppe mit 1 bis 6 Kohlenstoffatomen substituiert ist.

10. Verbindungen nach einem der vorhergehenden Ansprüche, **dadurch gekennzeichnet, dass** die Aralkylgruppen unter Benzyl oder Phenethyl ausgewählt sind, die gegebenenfalls substituiert sind mit mindestens einem Halogenatom, einer Alkylgruppe mit 1 bis 6 Kohlenstoffatomen, einer Hydroxygruppe, einer Alkoxygruppe mit 1 bis 3 Kohlenstoffatomen, einer Nitrofunktion, einer Polyethergruppe oder einer Aminogruppe, die gegebenenfalls mit Acetyl geschützt ist oder gegebenenfalls mit mindestens einer Alkylgruppe mit 1 bis 6 Kohlenstoffatomen oder einer Alkoxygruppe mit 1 bis 6 Kohlenstoffatomen substituiert ist.

11. Verbindungen nach einem der vorhergehenden Ansprüche, **dadurch gekennzeichnet, dass** die Alkenylgruppen unter den Gruppen ausgewählt sind, die vorzugsweise 2 bis 5 Kohlenstoffatome aufweisen und eine oder mehrere ethylenisch ungesättigte Bindungen besitzen.

12. Verbindungen nach einem der vorhergehenden Ansprüche, **dadurch gekennzeichnet, dass** der Zuckerrest unter den Resten ausgewählt ist, die von Glucose, Galactose, Mannose oder auch Glucuronsäure abgeleitet sind.

13. Verbindungen nach einem der vorhergehenden Ansprüche, **dadurch gekennzeichnet, dass** der Aminosäurerest unter den Resten ausgewählt ist, die von Lysin, Glycin oder Asparaginsäure abgeleitet sind.

14. Verbindungen nach einem der vorhergehenden Ansprüche, **dadurch gekennzeichnet, dass** der Aminosäurerest unter den Dipeptidresten oder Tripeptidresten ausgewählt ist, die durch die Kombination von Aminosäuren gebildet werden.

15. Verbindungen der Formel (I) nach einem der vorhergehenden Ansprüche, die ausgewählt sind unter:
1. 4-[2-(3-tert-Butyl-4-diethylamino-phenyl)-2-hydroxyimino-ethoxy-benzoesäure;
2. 4-[2-(3-tert-butyl-4-diethylamino-phenyl)-2-hydroxyimino-ethoxy]-2-hydroxybenzoesäure;
3. 4-[2-(3-tert-Butyl-4-pyrrolidin-1-yl-phenyl)-2-hydroxy-imino-ethoxy]-benzoesäure;
4. 4-[2-(3-tert-Butyl-4-pyrrolidin-1-yl-phenyl)-2-hydroxy-imino-ethoxy]-2-hydroxy-benzoesäure;
5. 4-{2-[4-(Acetyl-ethyl-amino)-3-tert-butyl-phenyl]-2-hydroxy-imino-ethoxy}-benzoesäure;
6. 4-{2-[4-(Acetyl-ethyl-amino)-3-tert-butyl-phenyl]-2-hydroxy-imino-ethoxy}-2-hydroxy-benzoesäure;
7. Ethyl-4-[2-(3-tert-butyl-4-diethylamino-phenyl)-2-hydroxy-imino-ethoxy]-2-hydroxy-benzoat;
8. Isopropyl-4-[2-(3-tert-butyl-4-diethylamino-phenyl)-2-hydroxyimino-ethoxy]-2-hydroxy-benzoat;
9. Isobutyl-4-[2-(3-tert-butyl-4-diethylamino-phenyl)-2-hydroxyimino-ethoxy]-2-hydroxy-benzoat;
10. Octyl-4-[2-(3-tert-butyl-4-diethylamino-phenyl)-2-hydroxyimino-ethoxy]-2-hydroxy-benzoat;
11. Dodecyl-4-[2-(3-tert-butyl-4-diethylamino-phenyl)-2-hydroxyimino-ethoxy]-2-hydroxy-benzoat;
12. 1-(3-tert-Butyl-4-diethylamino-phenyl)-2-[3-hydroxy-4-(morpholin-4-carbonyl)-phenoxy]-ethanon-oxim;
13. 4-[2-(3-tert-Butyl-4-diethylamino-phenyl)-2-hydroxyimino-ethoxy]-N-ethyl-2-hydroxy-N- methyl-benzamid;
14. 4-[2-(3-tert-Butyl-4-diethylamino-phenyl)-2-hydroxyimino-ethylsulfanyl]-2-hydroxy-benzoesäure;
15. 4-[2-(3-tert-Butyl-4-diethylamino-phenyl)-2-hydroxyimino-ethylsulfanyl]-benzoesäure;
16. 4-[2-(3-tert Butyl-4-diethylamino-phenyl)-2-hydroxyimino-ethylamino]-2-hydroxybenzoesäure;
17. 4-{[2-(3-tert-Butyl-4-diethylamino-phenyl)-2-hydroxyimino-ethyl]-methyl-amino}-2-hydroxy-benzoesäure;
18. 4-[3-(3-tert-Butyl-4-diethylamino-phenyl)-3-hydroxyimino-propyl]-2-hydroxy-benzoesäure;
19. 4-[2-(3-tert-Butyl-4-diethylamino-phenyl)-2-hydroxyimino-acetylamino]-2-hydroxy-benzoesäure;
20. 4-[2-(3-tert-Butyl-4-diethylamino-phenyl)-2-hydroxyimino-acetylamino]-benzoesäure;
21. 4-[2-(3-tert-Butyl-4-diethylamino-phenyl)-2-methoxyimino-acetylamino]-2-hydroxy-benzoesäure;
22. 4-[2-(3-tert-Butyl-4-diethylamino-phenyl)-2-methoxyimino-ethoxy]-2-hydroxy-benzoesäure;
23. 4-[2-(3-tert-Butyl-5-chlor-4-diethylamino-phenyl)-2-hydroxyimino-ethoxy]-2-hydroxy-benzoesäure;
24. 4-[2-(3-tert-Butyl-4-diethylamino-5-fluor-phenyl)-2-hydroxyimino-ethoxy]-2-hydroxy-benzoesäure;
25. 4-[2-(3-tert-Butyl-4-diethylamino-5-trifluormethyl-phenyl)-2-hydroxyimino-ethoxy]-2-hydroxy-benzoesäure;
26. 4-[2-(3-tert-Butyl-4-diethylamino-5-methyl-phenyl)-2-hydroxyimino-ethoxy]-2-hydroxy-benzoesäure;
27. 4-[2-(3-tert-Butyl-4-diethylamino-5-ethyl-phenyl)-2-hydroxyimino-ethoxy]-2-hydroxy-benzoesäure;
28. 4-{2-[3-tert-Butyl-4-diethylamino-5-(2-ethoxy-ethoxy)-phenyl]-2-hydroxyimino-ethoxy}-2-hydroxy-benzoesäure;
29. 4-[2-(3-tert-Butyl-4-diethylamino-5-propoxy-phenyl)-2-hydroxyimino-ethoxy]-2-hydroxy-benzoesäure;
30. 4-[2-(4-Diethylamino-3-isopropyl-phenyl)-2-hydroxyimino-ethoxy]-2-hydroxy-benzoesäure;
31. 4-[2-(4-Diethylamino-3-trifluormethyl-phenyl)-2-hydroxyimino-ethoxy]-2-hydroxy-benzoesäure;
32. 4-[2-(4-Diethylamino-phenyl)-2-hydroxyimino-ethoxy]-2-hydroxy-benzoesäure;
33. 4-{2-[3-tert-Butyl-4-(ethyl-methyl-amino)-phenyl]-2-hydroxyimino-ethoxy}-2-hydroxy-benzoesäure;
34. 4-[2-(3-tert-Butyl-4-dimethylamino-phenyl)-2-hydroxyimino-ethoxy]-2-hydroxy-benzoesäure;
35. 4-{2-(3-tert-Butyl-4-(ethyl-isobutyl-amino)-phenyl]-2-hydroxyimino-ethoxy}-2-hydroxy-benzoesäure;
36. 4-[2-(3-tert-Butyl-4-morpholin-4-yl-phenyl)-2-hydroxyimino-ethoxy]-2-hydroxy-benzoesäure;
37. 4-[2-(3-tert-Butyl-4-piperidin-1-yl-phenyl)-2-hydroxyimino-ethoxy]-2-hydroxy-benzoesäure;
38. 4-{2-[3-tert-Butyl-4-(2-oxo-piperidin-1-yl)-phenyl]-2-hydroxyimino-ethoxy}-benzoesäure;
39. 4-{2-[3-tert-Butyl-4-(2-oxo-pyrrolidin-1-yl)-phenyl]-2-hydroxyimino-ethoxy}-benzoesäure;
40. 4-[2-(3-tert-Butyl-4-ethylamino-phenyl)-2-hydroxyimino-ethoxy]-2-hydroxy-benzoesäure;
41. 4-{2-[4-(Ethyl-propionyl-amino)-3-tert-butyl-phenyl]-2-hydroxyimino-ethoxy}-2-hydroxy-benzoesäure;
42. 4-{2-[3-tert-Butyl-4-(ethoxycarbonyl-ethyl-amino)-phenyl]-2-hydroxyimino-ethoxy}-2-hydroxy-benzoesäure;
43. 4-{2-[3-tert-Butyl-4-(ethyl-methoxycarbonyl-amino)-phenyl]-2-hydroxyimino-ethoxy}-2-hydroxy-benzoesäure;
44. 4-{2-[4-(Ethyl-trifluoracetyl-amino)-3-tert-butyl-phenyl]-2-hydroxyimino-ethoxy}-2-hydroxy-benzoesäure;
45. 4-{2-[4-(Trifluoracetyl-amino)-3-tert butyl-phenyl]-2-hydroxyimino-ethoxy}-2- hydroxy-benzoesäure;
46. 4-{2-[3-tert-Butyl-4-(ethyl-isopropyl-amino)-phenyl]-2-hydroxyimino-ethoxy}-2-hydroxy-benzoesäure.

16. Verbindungen nach Anspruch 1 oder 2, **dadurch gekennzeichnet, dass** sie eines der folgenden Merkmale aufweisen:
- R 1 bedeutet die Gruppe -OR7;
- R2 bedeutet ein Wasserstoffatom oder -OH;
- R5, R6, die gleich oder verschieden sind, bedeuten Methyl oder Ethyl oder bilden gemeinsam eine Pyrrolidingruppe;
- Q bedeutet eine Gruppe -OR13.

17. Verbindungen nach einem der Ansprüche 1 bis 16 als Arzneimittel.

18. Verwendung einer Verbindung nach einem der Ansprüche 1 bis 16 zur Herstellung einer Zusammensetzung, die vorgesehen ist für die Behandlung von:
- dermatologischen Erkrankungen, die mit einer Verhornungsstörung verbunden sind, die auf der Differenzierung und Proliferation der Zellen beruht;
- Ichtyosis, ichtyosisartigen Zuständen, der Darier Krankheit, Palmoplantarkeratosen, Leukoplakien und leucoplakieformen Zuständen und Lichen der Haut oder der Schleimhäute (buccal);
- dermatologischen Erkrankungen mit entzündlicher immunoallergischer Komponente, mit oder ohne einer Störung der Proliferation der Zellen;
- Proliferationen der Dermis oder Epidermis, die gutartig oder bösartig und gegebenenfalls viralen Ursprungs sein können;
- Proliferationen, die von UV-Strahlung induziert sein können;
- präkanzerösen Hautläsionen;
- Immundermatosen;
- bullösen Immunerkrankungen;
- Kollagenerkrankungen;
- dermatologischen Erkrankungen mit immunologischer Komponente;
- ophthalmologischen Störungen;
- Stigmen epidermaler und/oder dermaler Atrophie, die durch lokale oder systemische Corticosteroide hervorgerufen werden, oder aller anderen Formen der Atrophie der Haut;
- Hauterkrankungen viralen Ursprungs;
- Hautstörungen, die mit einer UV-Exposition zusammenhängen; altersbedingter oder lichtinduzierter Hautalterung, aktionischen Pigmentierungen und Keratosen;
- Pathologien, die mit der altersbedingten oder aktinischen Hautalterung einhergehen;
- Störungen der Talgdrüsenfunktion;
- Störungen der Wundheilung oder Streifen; oder
- Pigmentierungsstörungen.

19. Pharmazeutische Zusammensetzung, **dadurch gekennzeichnet, dass** sie in einem physiologisch akzeptablen Trägerstoff mindestens eine der Verbindungen nach einem der Ansprüche 1 bis 16 enthält.

20. Zusammensetzung nach Anspruch 19, **dadurch gekennzeichnet, dass** die Konzentration der Verbindung(en) nach einem der Ansprüche 1 bis 16 im Bereich von 0,001 bis 10 Gew.-%, bezogen auf das Gesamtgewicht der Zusammensetzung, liegt.

21. Zusammensetzung nach Anspruch 20, **dadurch gekennzeichnet, dass** die Konzentration der Verbindung(en) nach einem der Ansprüche 1 bis 16 im Bereich von 0,01 bis 1 Gew.-%, bezogen auf das Gesamtgewicht der Zusammensetzung, liegt.

22. Kosmetische Zusammensetzung, **dadurch gekennzeichnet, dass** sie in einem physiologisch akzeptablen Träger mindestens eine Verbindung nach einem der Ansprüche 1 bis 16 enthält.

23. Zusammensetzung nach Anspruch 22, **dadurch gekennzeichnet, dass** die Konzentrationen der Verbindung(en) nach einem der Ansprüche 1 bis 16 im Bereich von 0,001 bis 3 Gew.-%, bezogen auf das Gesamtgewicht der Zusammensetzung, liegt.

24. Nichttherapeutische Verwendung einer kosmetischen Zusammensetzung, wie sie in einem der Ansprüche 22 oder 23 definiert ist, zur Vorbeugung und/ oder Behandlung der Alterszeichen und/oder trockener Haut.

25. Nichttherapeutische Verwendung einer kosmetischen Zusammensetzung, wie sie in einem der Ansprüche 22 oder 23 definiert ist, zur Körper- oder Haarpflege.

26. Kosmetisches Verfahren zur Verschönerung der Haut, **dadurch gekennzeichnet, dass** eine Zusammensetzung, wie sie in einem der Ansprüche 22 oder 23 definiert ist, auf die Haut aufgetragen wird.
